# EUROPEAN PATENT SPECIFICATION

(11) **EP 2 892 532 B1**
(45) Date of publication and mention of the grant of the patent: **13.02.2019**
(21) Application number: 13765886.0
(22) Date of filing: 09.09.2013
(51) Int. Cl.: A61K 31/47, A61P 35/00

(54) **INHIBITORS OF MET, VEGFR AND RET FOR USE IN THE TREATMENT OF LUNG ADENOCARCINOMA**
HEMMER VON MET, VEGFR UND RET ZUR VERWENDUNG IN DER BEHANDLUNG VON LUNGENADENOCARZINOM
INHIBITEURS DE MET, VEGFR ET RET POUR L'UTILISATION DANS LE TRAITEMENT DE L'ADÉNOCARCINOME PULMONAIRE

(30) Priority: 07.09.2012 US 201261698143 P
(43) Date of publication of application: 15.07.2015
(73) Proprietor: Exelixis, Inc., Alameda, CA 94502 (US)
(72) Inventor: AFTAB, Dana, T., San Rafael, CA 94903 (US)
(74) Representative: Simpson, Tobias Rutger
(86) International application number: PCT/US2013/058768
(87) International publication number: WO 2014/039971

(56) References cited:
- WO-A1-2013/066047
- WO-A1-2013/163428
- US-A1- 2012 070 368
- F. M. YAKES ET AL: "Cabozantinib (XL184), a Novel MET and VEGFR2 Inhibitor, Simultaneously Suppresses Metastasis, Angiogenesis, and Tumor Growth", MOLECULAR CANCER THERAPEUTICS, vol. 10, no. 12, 16 September 2011 (2011-09-16), pages 2298-2308, XP055031202, ISSN: 1535-7163, DOI: 10.1158/1535-7163.MCT-11-0264
- SCHOFFSKI P ET AL: "371 Phase 2 randomized discontinuation trial (RDT) of XL184 in patients (pts) with advanced solid tumors", EUROPEAN JOURNAL OF CANCER. SUPPLEMENT, vol. 8, no. 7, 1 November 2010 (2010-11-01), page 117, XP027498061, PERGAMON, OXFORD, GB ISSN: 1359-6349, DOI: 10.1016/S1359-6349(10)72078-4 [retrieved on 2010-11-01]
- WAKELEE H. A. ET AL.: "A phase Ib/II study of XL184 (BMS 907351) with and without erlotinib (E) in patients (pts) with non-small cell lung cancer (NSCLC)", JOURNAL OF CLINICAL ONCOLOGY , vol. 28, no. 15s, 3017, 2010, XP002714870, Meeting Library ASCO University Retrieved from the Internet: URL:http://meetinglibrary.asco.org/print/5 77140 [retrieved on 2013-10-14]

### SEQUENCE LISTING

<110> Exelixis, Inc.
<120> Method of Treating Lung Adenocarcinoma
<130> 224990/12-001C-US/369439
<140> PCT/US2013/058768
   <141> 2013-09-09
<150> US 61/698,143
   <151> 2012-09-07
<160> 8
<170> PatentIn version 3.5
<210> 1
   <211> 22
   <212> DNA
   <213> Artificial Sequence
<220>
   <223> Synthetically generated sequence
<400> 1
   ggcatttgac ttggtggtag at 22
<210> 2
   <211> 20
   <212> DNA
   <213> Artificial Sequence
<220>
   <223> Synthetically generated sequence
<400> 2
   tccaaattcg ccttctccta 20
<210> 3
   <211> 20
   <212> DNA
   <213> Artificial sequence
<220>
   <223> Synthetically generated sequence
<400> 3
   cctgggaacc cacagtcaag 20
<210> 4
   <211> 22
   <212> DNA
   <213> Artificial Sequence
<220>
   <223> Synthetically generated sequence
<400> 4
   attaggtggc aactgtagaa cc 22
<210> 5
   <211> 20
   <212> DNA
   <213> Artificial Sequence
<220>
   <223> Synthetically generated sequence
<400> 5
   agccacagat caggaaaaga 20
<210> 6
   <211> 20
   <212> DNA
   <213> Artificial Sequence
<220>
   <223> Synthetically generated sequence
<400> 6
   aggaaatgac caaccaccag 20
<210> 7
   <211> 20
   <212> DNA
   <213> Artificial Sequence
<220>
   <223> Synthetically generated sequence
<400> 7
   ccaaggtcat ccatgacaac 20
<210> 8
   <211> 19
   <212> DNA
   <213> Artificial Sequence
<220>
   <223> Synthetically generated sequence
<400> 8
   caccctgttg ctgtagcca 19

## Description

### Field of the Invention

This invention is directed to the detection, diagnosis and treatment of cancer, particularly lung adenocarcinoma, using an inhibitor of MET, VEGFR, and RET.

### Background of the Invention

Lung cancer is the leading cause of cancer-related mortality worldwide. Recent developments in targeted therapies have led to a treatment paradigm shift in non-small-cell lung cancer (NSCLC). Mok TS, Wu YL, Thongprasert S, Yang CH, Chu DT, Saijo N, Sunpaweravong P, Han B, Margono B, Ichinose Y, Nishiwaki Y, Ohe Y, Yang JJ, Chewaskulyong B, Jiang H, Duffield EL, Watkins CL, Armour AA, Fukuoka M. Gefitinib or carboplatin-paclitaxel in pulmonary adenocarcinoma. N Engl J Med. 2009 Sep 3;361(10):947-57.Maemondo M, Inoue A, Kobayashi K, Sugawara S, Oizumi S, Isobe H, Gemma A, Harada M, Yoshizawa H, Kinoshita I, Fujita Y, Okinaga S, Hirano H, Yoshimori K, Harada T, Ogura T, Ando M, Miyazawa H, Tanaka T, Saijo Y, Hagiwara K, Morita S, Nukiwa T; North-East Japan Study Group. Gefitinib or chemotherapy for non-small-cell lung cancer with mutated EGFR. N Engl J Med. 2010 Jun 24;362(25):2380-8. Epidermal growth factor receptor (EGFR) tyrosine kinase inhibitors (TKIs), gefitinib and erlotinib, and the anaplastic lymphoma kinase (ALK) TKI, crizotinib, have shown clinical activity in NSCLC patients with *EGFR* mutations or *ALK* gene rearrangements. Kwak EL, Bang YJ, Camidge DR, Shaw AT, Solomon B, Maki RG, Ou SH, Dezube BJ, Jänne PA, Costa DB, Varella-Garcia M, Kim WH, Lynch TJ, Fidias P, Stubbs H, Engelman JA, Sequist LV, Tan W, Gandhi L, Mino-Kenudson M, Wei GC, Shreeve SM, Ratain MJ, Settleman J, Christensen JG, Haber DA, Wilner K, Salgia R, Shapiro GI, Clark JW, Iafrate AJ. Anaplastic lymphoma kinase inhibition in non-small-cell lung cancer. N Engl J Med. 2010 Oct 28;363(18):1693-703. In addition, *ROSI* gene rearrangement has been reported in approximately 2% of patients with NSCLC, and clinical activity has been reported using crizotinib in this patient subgroup. Bergethon K, Shaw AT, Ou SH, Katayama R, Lovly CM, McDonald NT, Massion PP, Siwak-Tapp C, Gonzalez A, Fang R, Mark EJ, Batten JM, Chen H, Wilner KD, Kwak EL, Clark JW, Carbone DP, Ji H, Engelman JA, Mino-Kenudson M, Pao W, Iafrate AJ. ROS1 rearrangements define a unique molecular class of lung cancers. J Clin Oncol. 2012 Mar 10;30(8):863-70. Shaw AT, Camidge, Engelman JA, Solomon BJ, Kwak EL, Clark JW, Salgia R, Shapiro, Bang YJ, Tan W, Tye L, Wilner KD, Stephenson P, Varella-Garcia M, Bergethon K, Iafrate AJ, Ou SH. Clinical activity of crizotinib in advanced non-small cell lung cancer (NSCLC) harboring ROSI gene rearrangement. J Clin Oncol. 2012 30 (suppl; abstr 7508). Fusion of the *KJF5B* (the kinesin family 5B) gene and the *RET* oncogene has been recently reported as a driver mutation in 1-2% of NSCLC patients and are a focus as a therapeutic target. Kohno T, Ichikawa H, Totoki Y, Yasuda K, Hiramoto M, Nammo T, Sakamoto H, Tsuta K, Furuta K, Shimada Y, Iwakawa R, Ogiwara H, Oike T, Enari M, Schetter AJ, Okayama H, Haugen A, Skaug V, Chiku S, Yamanaka I, Arai Y, Watanabe S, Sekine I, Ogawa S, Harris CC, Tsuda H, Yoshida T, Yokota J, Shibata T. KIF5B-RET fusions in lung adenocarcinoma. Nat Med. 2012 Feb 12;18(3):375-7. Takeuchi K, Soda M, Togashi Y, Suzuki R, Sakata S, Hatano S, Asaka R, Hamanaka W, Ninomiya H, Uehara H, Lim Choi Y, Satoh Y, Okumura S, Nakagawa K, Mano H, Ishikawa Y. RET, ROSI and ALK fusions in lung cancer. Nat Med. 2012 Feb 12;18(3):378-81. Lipson D, Capelletti M, Yelensky R, Otto G, Parker A, Jarosz M, Curran JA, Balasubramanian S, Bloom T, Brennan KW, Donahue A, Downing SR, Frampton GM, Garcia L, Juhn F, Mitchell KC, White E, White J, Zwirko Z, Peretz T, Nechushtan H, Soussan-Gutman L, Kim J, Sasaki H, Kim HR, Park SI, Ercan D, Sheehan CE, Ross JS, Cronin MT, Jänne PA, Stephens PJ. Identification of new ALK and RET gene fusions from colorectal and lung cancer biopsies. Nat Med. 2012 Feb 12;18(3):382-4. Thus, it is becoming more important to identify key driver genes in NSCLC and to develop therapies for each genomic subset of patients.

### Summary of the Invention

These and other needs are met by the present invention which is directed to a method for treating lung adenocarcinoma using an inhibitor of MET, VEGFR, and RET. The method comprises administering a therapeutically effective amount of a compound that modulates MET, VEGFR, and RET to a patient in need of such treatment. In one embodiment, the lung adenocarcinoma is non-small cell lung cancer (NSCLC). More particularly, the lung adenocarcinoma is most frequently *KIF5B-RET* fusion-positive NSCLC, and other known RET fusions including CCDC6, NCOA4, and TRIM33, and other RET fusions on chromosome 10.

In one aspect, the present invention is directed to a method for treating NSCLC in a patient in need of such treatment, comprising administering a therapeutically effective amount of a compound that simultaneously modulates MET, VEGFR, and RET to the patient.

In one embodiment of this and other aspects, the dual acting MET/VEGFR/RET inhibitor is a compound of Formula I 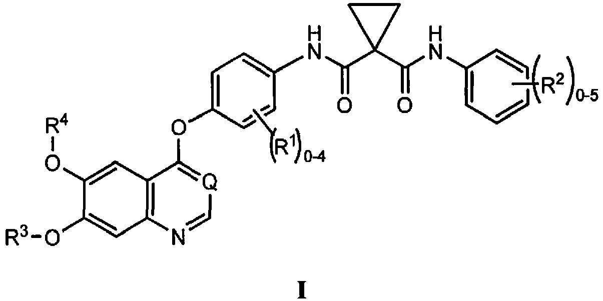 or a pharmaceutically acceptable salt thereof, wherein:
R¹ is halo;
R² is halo;
R³ is (C₁-C₆)alkyl;
R⁴ is (C₁-C₆)alkyl; and
Q is CH or N.

In another embodiment, the compound of Formula I is a compound of Formula Ia 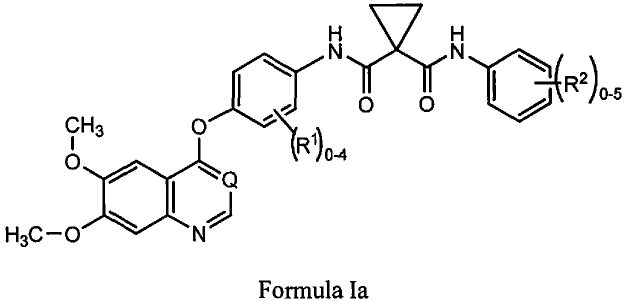 or a pharmaceutically acceptable salt thereof, wherein:
R¹ is halo;
R² is halo; and
Q is CH orN.

In another embodiment, the compound of Formula I is compound 1: 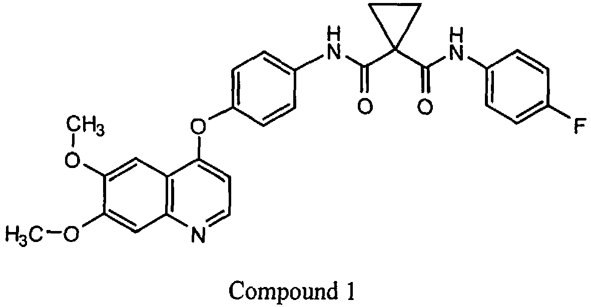 or a pharmaceutically acceptable salt thereof. Compound 1 is known as N-(4-{[6,7-bis(methyloxy)quinolin-4-yl]oxy}phenyl)-N'-(4-fluorophenyl)cyclopropane-1,1-dicarboxamide and by the name Cabozantinib.

Compound 1 is a potent inhibitor of c-MET, RET, and VEGFR2. Yakes FM, Chen J, Tan J, Yamaguchi K, Shi Y, Yu P, Qian F, Chu F, Bentzien F, Cancilla B, Orf J, You A, Laird AD, Engst S, Lee L, Lesch J, Chou YC, Joly AH. Cabozantinib (XL184), a novel MET and VEGFR2 inhibitor, simultaneously suppresses metastasis, angiogenesis, and tumor growth. Mol Cancer Ther. 2011 Dec;10(12):2298-308. In preclinical studies, Compound 1-mediated inhibition of kinase activity produced rapid and robust regression of tumor vasculature, tumor invasiveness and metastasis, and prolonged survival. Sennino B. Inhibition of tumor invasiveness by c-MET/VEGFR blockade. Presented at: Gordon Research Conference: Angiogenesis; August 2-7, 2009; Newport, RI. You WK, Falcon B, Hashizume H et al. Exaggerated regression of blood vessels, hypoxia, and apoptosis in tumors after c-MET and VEGFR inhibition. Am J Pathol, submitted.

In another embodiment, the compound of Formula I, Ia, or Compound 1 is administered as a pharmaceutical composition comprising a pharmaceutically acceptable additive, diluent, or excipient.

In another aspect, the invention provides a method for detecting, diagnosing and treating *KIF5B-RET* fusion-positive NSCLC, and other known RET fusions including CCDC6, NCOA4, and TRIM33, and and other RET fusions on chromosome 10 (See Cancer Discovery, Alexander Drilon, Lu Wang, Adnan Hasanovic, et al., Published OnlineFirst March 26, 2013; DOI: 10.1158/2159-8290.CD-13-0035, referring to American Association for Cancer Research, June 2013) comprising administering a therapeutically effective amount of a pharmaceutical composition comprising a Compound of Formula I or the malate salt of a Compound of Formula I or another pharmaceutically acceptable salt of a Compound of Formula I, to a patient in need of such treatment. In a specific embodiment, the Compound of Formula I is Compound 1 or the malate salt of Compound 1.

In another aspect, the invention provides a method for treating a lung adenocarcinoma which is *KIF5B-RET* fusion positive non-small cell lung cancer in a patient in need of such treatment, comprising administering to the patient an effective amount of compound 1: 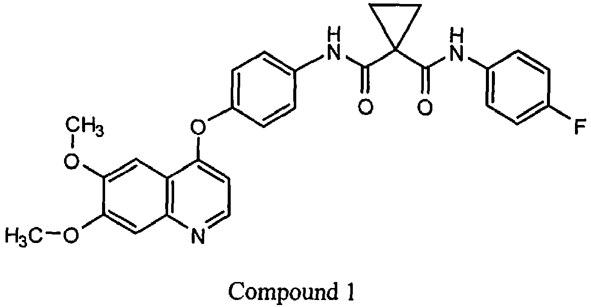 or a pharmaceutically acceptable salt thereof.

### Brief Description of the Figures

Figure 1A depicts inhibition of phosphorylation of RET in vivo in TT-tumor-bearing animals that were ad3ministered single escalating doses of Compound 1 or water vehicle.
Figure 1B depicts the effect of the administration of a single oral dose of Compound 1 (100 mg/kg) on mice bearing TT tumors on phosphorylation levels and total RET, AKT, and ERK in tumor lysates.
Figure 1C provides densitometric quantitation of the duration of inhibition of phosphorylation of RET versus plasma concentrations of Compound 1, along with representative Western blot images.
Figure 2A shows that Compound 1 inhibits TT xenograft tumor growth that correlating with serum reductions in calcitonin in *nu*/*nu* mice bearing TT tumors that were orally administered once daily water vehicle (□) or cabozantinib at 3 mg/kg (∇), 10 mg/kg (○), 30 mg/kg (◆), or 60 mg/kg (◇) for 21 days.
Figure 2B shows circulating calcitonin levels determined in serum preparations from whole blood collected after the final indicated doses.
Figure 3 depicts the response of a patient with *KIF5B-RET* fusion-positive NCSLC to Compound 1. Computed tomography scans of the chest were obtained at baseline (Figure 1A) and after 9 weeks (Figure 1B) of Compound 1.
Figure 4A depicts KIF5B-RET genome PCR and Sanger sequencing from pre- and post-treatment tumor samples.
Figure 4B depicts KIF5B-RET RT-PCR and Sanger sequencing from post-treatment tumor sample.
Figure 4C depicts break-apart FISH at the *RET* locus in tumor cells.

### Detailed Description of the Invention

### Abbreviations and Definitions

The following abbreviations and terms have the indicated meanings throughout this application.

| **Abbreviation** | **Meaning** |
|---|---|
| Ac | Acetyl |
| Br | Broad |
| °C | Degrees Celsius |
| c- | Cyclo |
| CBZ | CarboBenZoxy = benzyloxycarbonyl |
| d | Doublet |
| dd | Doublet of doublet |
| dt | Doublet of triplet |
| DCM | Dichloromethane |
| DME | 1,2-dimethoxyethane |
| DMF | *N,N*-Dimethylformamide |
| DMSO | dimethyl sulfoxide |
| EI | Electron Impact ionization |
| G | Gram(s) |
| h or hr | Hour(s) |
| HPLC | High pressure liquid chromatography |
| L | Liter(s) |
| M | Molar or molarity |
| m | Multiplet |
| Mg | Milligram(s) |
| MHz | Megahertz (frequency) |
| Min | Minute(s) |
| mL | Milliliter(s) |
| µL | Microliter(s) |
| µM | Micromole(s) or micromolar |
| mM | Millimolar |
| Mmol | Millimole(s) |
| Mol | Mole(s) |
| MS | Mass spectral analysis |
| N | Normal or normality |
| nM | Nanomolar |
| NMR | Nuclear magnetic resonance spectroscopy |
| q | Quartet |
| RT | Room temperature |
| s | Singlet |
| t or tr | Triplet |
| TFA | Trifluoroacetic acid |
| THF | Tetrahydrofuran |
| TLC | Thin layer chromatography |

The symbol "-" means a single bond, "=" means a double bond.

When chemical structures are depicted or described, unless explicitly stated otherwise, all carbons are assumed to have hydrogen substitution to conform to a valence of four. For example, in the structure on the left-hand side of the schematic below there are nine hydrogens implied. The nine hydrogens are depicted in the right-hand structure. Sometimes a particular atom in a structure is described in textual formula as having a hydrogen or hydrogens as substitution (expressly defined hydrogen), for example, -CH₂CH₂-. It is understood by one of ordinary skill in the art that the aforementioned descriptive techniques are common in the chemical arts to provide brevity and simplicity to description of otherwise complex structures.

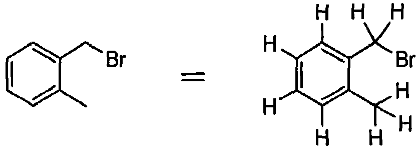

If a group "R" is depicted as "floating" on a ring system, as for example in the formula: 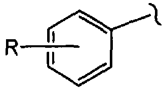 then, unless otherwise defined, a substituent "R" may reside on any atom of the ring system, assuming replacement of a depicted, implied, or expressly defined hydrogen from one of the ring atoms, so long as a stable structure is formed.

If a group "R" is depicted as floating on a fused ring system, as for example in the formulae: 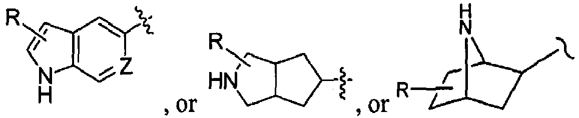 then, unless otherwise defined, a substituent "R" may reside on any atom of the fused ring system, assuming replacement of a depicted hydrogen (for example the -NH- in the formula above), implied hydrogen (for example as in the formula above, where the hydrogens are not shown but understood to be present), or expressly defined hydrogen (for example where in the formula above, "Z" equals =CH-) from one of the ring atoms, so long as a stable structure is formed. In the example depicted, the "R" group may reside on either the 5-membered or the 6-membered ring of the fused ring system. When a group "R" is depicted as existing on a ring system containing saturated carbons, as for example in the formula: 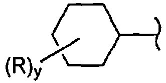 where, in this example, "y" can be more than one, assuming each replaces a currently depicted, implied, or expressly defined hydrogen on the ring; then, unless otherwise defined, where the resulting structure is stable, two "R's" may reside on the same carbon. A simple example is when R is a methyl group; there can exist a geminal dimethyl on a carbon of the depicted ring (an "annular" carbon). In another example, two R's on the same carbon, including that carbon, may form a ring, thus creating a spirocyclic ring (a "spirocyclyl" group) structure with the depicted ring as for example in the formula:

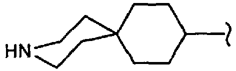

"Halogen" or "halo" refers to fluorine, chlorine, bromine or iodine.

"KIF5B" may refer to the KIF5B protein, or the KIF5B gene. The KIF5B protein, which is also called as Kinesin-1 heavy chain, is a protein encoded by KIF5B gene. The KIF5B protein may be derived from a mammal, such as a human. The human KIF5B gene encoding the human KIF5B protein is localized to chromosome 10 (10q11.22) and contains 26 exons. KIF5B is further described herein.

"KIF5B-RET fusion - protein or gene" refers to a fusion protein including N-terminal domain of a fusion partner, such as KIF5B and the C-terminal domain of RET protein. The N-terminal domain of a fusion partner may be positioned at N-terminus of the fusion protein, and the C-terminal domain of RET protein may be positioned at C-terminus of the fusion protein. The fusion partner may be a N-terminal domain of KIF5B protein, which is positioned at N-terminus of the fusion protein. In this case, the fusion protein may be represented as KIF5B-RET protein which includes N-terminal domain of KIF5B protein at N-terminus and C-terminal domain of RET protein at C-terminus. Another embodiment provides a fusion gene encoding the fusion protein, where a gene encoding the N-terminal domain of the fusion partner positions at 5' end and a gene encoding the C-terminal domain of the RET protein positions at 3' end.

"Patient" for the purposes of the present invention includes humans and other animals, particularly mammals, and other organisms. Thus the methods are applicable to both human therapy and veterinary applications. In another embodiment the patient is a mammal, and in another embodiment the patient is human.

"Pharmaceutically acceptable salt" of a compound means a salt that is pharmaceutically acceptable and that possesses the desired pharmacological activity of the parent compound. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985, or S. M. Berge, et al., "Pharmaceutical Salts," J. Pharm. Sci., 1977;66: 1-19.

Examples of pharmaceutically acceptable acid addition salts include those formed with inorganic acids such as hydrochloric acid, hydrobromic acid, sulfuric acid, nitric acid, phosphoric acid, and the like; as well as organic acids such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, tartaric acid, malic acid, citric acid, benzoic acid, cinnamic acid, 3-(4-hydroxybenzoyl)benzoic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, p-toluenesulfonic acid, and salicylic acid and the like.

"Prodrug" refers to compounds that are transformed (typically rapidly) *in vivo* to yield the parent compound of the above formulae, for example, by hydrolysis in blood. Common examples include, but are not limited to, ester and amide forms of a compound having an active form bearing a carboxylic acid moiety. Examples of pharmaceutically acceptable esters of the compounds of this invention include, but are not limited to, alkyl esters (for example with between about one and about six carbons) the alkyl group is a straight or branched chain. Acceptable esters also include cycloalkyl esters and arylalkyl esters such as, but not limited to benzyl. Examples of pharmaceutically acceptable amides of the compounds of this invention include, but are not limited to, primary amides, and secondary and tertiary alkyl amides (for example with between about one and about six carbons). Amides and esters of the compounds of the present invention may be prepared according to conventional methods. A thorough discussion of prodrugs is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

"RET" or the "RET protein" is a transmembrane receptor tyrosine kinase, and is further described herein.

"Therapeutically effective amount" is an amount of a compound of the invention, that when administered to a patient, ameliorates a symptom of the disease. A therapeutically effective amount is intended to include an amount of a compound alone or in combination with other active ingredients effective to modulate c-Met, and/or VEGFR2, or effective to treat or prevent cancer. The amount of a compound of the invention which constitutes a "therapeutically effective amount" will vary depending on the compound, the disease state and its severity, the age of the patient to be treated, and the like. The therapeutically effective amount can be determined by one of ordinary skill in the art having regard to their knowledge and to this disclosure.

"Treating" or "treatment" of a disease, disorder, or syndrome, as used herein, includes (i) preventing the disease, disorder, or syndrome from occurring in a human, i.e. causing the clinical symptoms of the disease, disorder, or syndrome not to develop in an animal that may be exposed to or predisposed to the disease, disorder, or syndrome but does not yet experience or display symptoms of the disease, disorder, or syndrome; (ii) reversing or inhibiting the disease, disorder, or syndrome, i.e., arresting its development; and (iii) relieving the disease, disorder, or syndrome, i.e., causing regression of the disease, disorder, or syndrome. As is known in the art, adjustments for systemic versus localized delivery, age, body weight, general health, sex, diet, time of administration, drug interaction and the severity of the condition may be necessary, and will be ascertainable with routine experience.

"Yield" for each of the reactions described herein is expressed as a percentage of the theoretical yield.

### Embodiments

In one embodiment the compound of Formula I is the compound of Formula **Ia:** 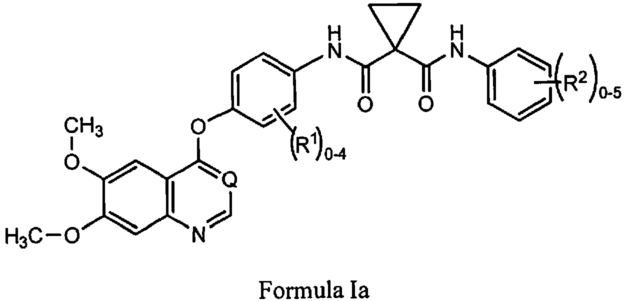 or a pharmaceutically acceptable salt thereof, wherein:
R¹ is halo;
R² is halo; and
Q is CH orN.

In another embodiment, the compound of Formula I is Compound 1: 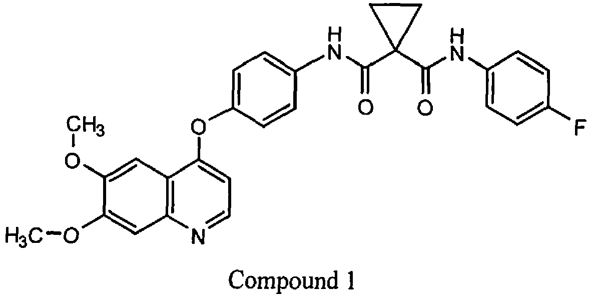 or a pharmaceutically acceptable salt thereof. As indicated previously, compound 1 is referred to herein as N-(4-{[6,7-bis(methyloxy)quinolin-4-yl]oxy}phenyl)-N'-(4-fluorophenyl)cyclopropane-1,1-dicarboxamide. WO2005/030140 discloses Compound 1 and describes how it is made (Example 12, 37, 38, and 48) and also discloses the therapeutic activity of this compound to inhibit, regulate and/or modulate the signal transduction of kinases, (Assays, Table 4, entry 289). Example 48 is on paragraph [0353] in WO 2005/030140.

In other embodiments, the compound of Formula I, la, or Compound 1, or a pharmaceutically acceptable salt thereof, is administered as a pharmaceutical composition, wherein the pharmaceutical composition additionally comprises a pharmaceutically acceptable carrier, excipient, or diluent. In a specific embodiment, the Compound of Formula I is Compound 1.

The compound of Formula I, Formula Ia and Compound I, as described herein, includes both the recited compounds as well as individual isomers and mixtures of isomers. In each instance, the compound of Formula I includes the pharmaceutically acceptable salts, hydrates, and/or solvates of the recited compounds and any individual isomers or mixture of isomers thereof.

In other embodiments, the compound of Formula I, la, or Compound 1 can be the (L)-malate salt. The malate salt of the Compound of Formula I and of Compound 1 is disclosed in PCT/US2010/021194 and USSN 61/325095.

In other embodiments, the compound of Formula I is the (D)-malate salt.

In other embodiments, the compound of Formula Ia is the malate salt.

In other embodiments, the compound of Formula Ia is the (L)-malate salt.

In other embodiments, Compound 1 is the (D)-malate salt.

In other embodiments, Compound 1 is the malate salt.

In other embodiments, Compound 1 is the (L)-malate salt.

In another embodiment, the malate salt is in the crystalline N-1 form or the N-2 form of the (L) malate salt and/or the (D) malate salt of Compound 1 as disclosed in United States patent Application Ser. No. 61/325095. *Also see* WO2008/083319 for the properties of crystalline enantiomers, including the N-1 and/or the N-2 crystalline forms of the malate salt of Compound 1. Methods of making and characterizing such forms are fully described in PCT/US10/021194.

In another embodiment, the invention is directed to a method for reversing or inhibiting NSCLC, comprising administering to a patient in need of such treatment a therapeutically effective amount of a compound of Formula I in any of the embodiments disclosed herein. In a specific embodiment, the Compound of Formula I is Compound 1.

In another embodiment, the invention is directed to a method for reversing or inhibiting *KIF5B-RET* fusion-positive NSCLC, comprising administering to a patient in need of such treatment a therapeutically effective amount of a compound of Formula I in any of the embodiments disclosed herein. In a specific embodiment, the Compound of Formula I is Compound 1.

In another embodiment, the compound of Formula I is administered before, concurrently, or subsequent to one or more other treatments. In another embodiment, the compound of Formula I is administered subsequent to one or more treatments. "Treatment" means any of the treatment options are available to the skilled artisan, including surgery, chemotherapeutic agents, hormone therapies, antibodies, immunotherapies, radioactive iodine therapy, and radiation. In particular, "treatment' means another chemotherapeutic agent or antibody.

Thus, in another embodiment, the compound of Formula I is administered post-cisplatin and/or gemcitabine treatment.

In another embodiment, the compound of Formula I is administered post-doectaxel treatment.

In another embodiment, the compound of Formula I is administered post HER-2 antibody treatment. In another embodiment, the HER-2 antibody is trastuzumab.

In another embodiment, the compound of Formula I is administered post-cisplatin and/or gemcitabine and/or docetaxel treatment.

In another embodiment, the Compound of Formula I, Ia, or Compound 1 or a pharmaceutically acceptable salt thereof is administered orally once daily as a tablet or capsule. In these and other embodiments, the Compound of Formula I is Compound 1.

In another embodiment, Compound 1 is administered orally as its free base or the malate salt as a capsule or tablet.

In another embodiment, Compound 1 is administered orally once daily as its free base or as the malate salt as a capsule or tablet containing up to 100 mg of Compound 1.

In another embodiment, Compound 1 is administered orally once daily as its free base or as the malate salt as a capsule or tablet containing 100 mg of Compound 1.

In another embodiment, Compound 1 is administered orally once daily as its free base or as the malate salt as a capsule or tablet containing 95 mg of Compound 1.

In another embodiment, Compound 1 is administered orally once daily as its free base or as the malate salt as a capsule or tablet containing 90 mg of Compound 1.

In another embodiment, Compound 1 is administered orally once daily as its free base or as the malate salt as a capsule or tablet containing 85 mg of Compound 1.

In another embodiment, Compound 1 is administered orally once daily as its free base or as the malate salt as a capsule or tablet containing 80 mg of Compound 1.

In another embodiment, Compound 1 is administered orally once daily as its free base or as the malate salt as a capsule or tablet containing 75 mg of Compound 1.

In another embodiment, Compound 1 is administered orally once daily as its free base or as the malate salt as a capsule or tablet containing 70 mg of Compound 1.

In another embodiment, Compound 1 is administered orally once daily as its free base or as the malate salt as a capsule or tablet containing 65 mg of Compound 1.

In another embodiment, Compound 1 is administered orally once daily as its free base or as the malate salt as a capsule or tablet containing 60 mg of Compound 1.

In another embodiment, Compound 1 is administered orally once daily as its free base or as the malate salt as a capsule or tablet containing 55 mg of Compound 1.

In another embodiment, Compound 1 is administered orally once daily as its free base or as the malate salt as a capsule or tablet containing 50 mg of Compound 1.

In another embodiment, Compound 1 is administered orally once daily as its free base or as the malate salt as a capsule or tablet containing 45 mg of Compound 1.

In another embodiment, Compound 1 is administered orally once daily as its free base or as the malate salt as a capsule or tablet containing 40 mg of Compound 1.

In another embodiment, Compound 1 is administered orally once daily as its free base or as the malate salt as a capsule or tablet containing 30 mg of Compound 1.

In another embodiment, Compound 1 is administered orally once daily as its free base or as the malate salt as a capsule or tablet containing 25 mg of Compound 1.

In another embodiment, Compound 1 is administered orally once daily as its free base or as the malate salt as a capsule or tablet containing 20 mg of Compound 1.

In another embodiment, Compound 1 is administered orally once daily as its free base or as the malate salt as a capsule or tablet containing 15 mg of Compound 1.

In another embodiment, Compound 1 is administered orally once daily as its free base or as the malate salt as a capsule or tablet containing 10 mg of Compound 1.

In another embodiment, Compound 1 is administered orally once daily as its free base or as the malate salt as a capsule or tablet containing 5 mg of Compound 1.

In another embodiment, Compound 1 is administered as its free base or malate salt orally once daily as a tablet as provided in the following table.

| **Ingredient** | **(% w/w)** |
|---|---|
| Compound 1 | 31.68 |
| Microcrystalline Cellulose | 38.85 |
| Lactose anhydrous | 19.42 |
| Hydroxypropyl Cellulose | 3.00 |
| Croscarmellose Sodium | 3.00 |
| **Total Intra-granular** | **95.95** |
| Silicon dioxide, Colloidal | 0.30 |
| Croscarmellose Sodium | 3.00 |
| Magnesium Stearate | 0.75 |
| **Total** | **100.00** |

In another embodiment, Compound 1 is administered orally as its free base or malate salt once daily as a tablet as provided in the following table.

| **Ingredient** | **(% w/w)** |
|---|---|
| Compound 1 | 25.0-33.3 |
| Microcrystalline Cellulose | q.s |
| Hydroxypropyl Cellulose | 3 |
| Poloxamer | 0-3 |
| Croscarmellose Sodium | 6.0 |
| Colloidal Silicon Dioxide | 0.5 |
| Magnesium Stearate | 0.5-1.0 |
| Total | 100 |

In another embodiment, Compound 1 is administered orally as its free base or malate salt once daily as a tablet as provided in the following table.

| **Ingredient** | **Theoretical Quantity (mg/unit dose)** |
|---|---|
| Compound 1 | 100.0 |
| Microcrystalline Cellulose PH-102 | 155.4 |
| Lactose Anhydrous 60M | 77.7 |
| Hydroxypropyl Cellulose, EXF | 12.0 |
| Croscarmellose Sodium | 24 |
| Colloidal Silicon Dioxide | 1.2 |
| Magnesium Stearate (Non-Bovine) | 3.0 |
| Opadry Yellow | 16.0 |
| **Total** | **416** |

Any of the tablet formulations provided above can be adjusted according to the dose of Compound 1 desired. Thus, the amount of each of the formulation ingredients can be proportionally adjusted to provide a table formulation containing various amounts of Compound 1 as provided in the previous paragraphs. In another embodiment, the formulations can contain 20, 40, 60, or 80 mg of Compound 1.

In these and other embodiments, the invention provides a method for inhibiting or reversing the progress of abnormal cell growth in a mammal, comprising administering Compound 1 or a pharmaceutically acceptable salt thereof, wherein the abnormal cell growth is cancer mediated by *KlF5B-RET.* In one embodiment, the cancer is lung adenocarcinoma. In another, the lung adenocarcinoma is non-small cell lung cancer. In another, the lung adenocarcinoma is *KIF5B-RET* fusion-positive non-small cell lung cancer. In another embodiment, Compound 1 or a pharmaceutically acceptable salt thereof is administered as a pharmaceutical composition comprising Compound 1 or a pharmaceutically acceptable salt thereof and at least one pharmaceutically acceptable carrier. In another embodiment, the compound of Formula I is administered subsequent to another form of treatment. In another embodiment, Compound 1 is administered post-cisplatin and/or gemcitabine treatment. In another embodiment, Compound 1 is administered post-doectaxel treatment. In another embodiment, Compound 1 is administered post-cisplatin and/or gemcitabine and/or docetaxel treatment.

### Administration

Administration of the compound of Formula I, Formula la, or Compound 1, or a pharmaceutically acceptable salt thereof, in pure form or in an appropriate pharmaceutical composition, can be carried out via any of the accepted modes of administration or agents for serving similar utilities. Thus, administration can be, for example, orally, nasally, parenterally (intravenous, intramuscular, or subcutaneous), topically, transdermally, intravaginally, intravesically, intracistemally, or rectally, in the form of solid, semi-solid, lyophilized powder, or liquid dosage forms, such as for example, tablets, suppositories, pills, soft elastic and hard gelatin dosages (which can be in capsules or tablets), powders, solutions, suspensions, or aerosols, or the like, specifically in unit dosage forms suitable for simple administration of precise dosages.

The compositions will include a conventional pharmaceutical carrier or excipient and a compound of Formula I as the/an active agent, and, in addition, may include carriers and adjuvants, etc.

Adjuvants include preserving, wetting, suspending, sweetening, flavoring, perfuming, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

If desired, a pharmaceutical composition of the compound of Formula I may also contain minor amounts of auxiliary substances such as wetting or emulsifying agents, pH buffering agents, antioxidants, and the like, such as, for example, citric acid, sorbitan monolaurate, triethanolamine oleate, butylalted hydroxytoluene, etc.

The choice of composition depends on various factors such as the mode of drug administration (e.g., for oral administration, compositions in the form of tablets, pills or capsules) and the bioavailability of the drug substance. Recently, pharmaceutical compositions have been developed especially for drugs that show poor bioavailability based upon the principle that bioavailability can be increased by increasing the surface area i.e., decreasing particle size. For example, U.S. Pat. No. 4,107,288 describes a pharmaceutical composition having particles in the size range from 10 to 1,000 nm in which the active material is supported on a crosslinked matrix of macromolecules. U.S. Pat. No. 5,145,684 describes the production of a pharmaceutical composition in which the drug substance is pulverized to nanoparticles (average particle size of 400 nm) in the presence of a surface modifier and then dispersed in a liquid medium to give a pharmaceutical composition that exhibits remarkably high bioavailability.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

One specific route of administration is oral, using a convenient daily dosage regimen that can be adjusted according to the degree of severity of the disease-state to be treated.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, cellulose derivatives, starch, alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscarmellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate and the like (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid dosage forms as described above can be prepared with coatings and shells, such as enteric coatings and others well known in the art. They may contain pacifying agents, and can also be of such composition that they release the active compound or compounds in a certain part of the intestinal tract in a delayed manner. Examples of embedded compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., the compound of Formula I, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administration are, for example, suppositories that can be prepared by mixing the compound of Formula I with, for example, suitable non-irritating excipients or carriers such as cocoa butter, polyethyleneglycol or a suppository wax, which are solid at ordinary temperatures but liquid at body temperature and therefore, melt while in a suitable body cavity and release the active component therein.

Dosage forms for topical administration of the compound of Formula I include ointments, powders, sprays, and inhalants. The active component is admixed under sterile conditions with a physiologically acceptable carrier and any preservatives, buffers, or propellants as may be required. Ophthalmic compositions, eye ointments, powders, and solutions are also contemplated as being within the scope of this disclosure.

Compressed gases may be used to disperse the compound of Formula I in aerosol form. Inert gases suitable for this purpose are nitrogen, carbon dioxide, etc.

Generally, depending on the intended mode of administration, the pharmaceutically acceptable compositions will contain about 1% to about 99% by weight of a compound(s) of Formula I, or a pharmaceutically acceptable salt thereof, and 99% to 1% by weight of a suitable pharmaceutical excipient. In one example, the composition will be between about 5% and about 75% by weight of a compound(s) of Formula I, Formula Ia, or Compound 1, or a pharmaceutically acceptable salt thereof, with the rest being suitable pharmaceutical excipients.

Actual methods of preparing such dosage forms are known, or will be apparent, to those skilled in this art; for example, see Remington's Pharmaceutical Sciences, 18th Ed., (Mack Publishing Company, Easton, Pa., 1990). The composition to be administered will, in any event, contain a therapeutically effective amount of a compound of Formula I, or a pharmaceutically acceptable salt thereof, for treatment of a disease-state in accordance with the teachings of this disclosure.

The compounds of this disclosure, or their pharmaceutically acceptable salts or solvates, are administered in a therapeutically effective amount which will vary depending upon a variety of factors including the activity of the specific compound employed, the metabolic stability and length of action of the compound, the age, body weight, general health, sex, diet, mode and time of administration, rate of excretion, drug combination, the severity of the particular disease-states, and the host undergoing therapy. The compound of Formula I, Formula la, or Compound 1, can be administered to a patient at dosage levels in the range of about 0.1 to about 1,000 mg per day. For a normal human adult having a body weight of about 70 kilograms, a dosage in the range of about 0.01 to about 100 mg per kilogram of body weight per day is an example. The specific dosage used, however, can vary. For example, the dosage can depend on a number of factors including the requirements of the patient, the severity of the condition being treated, and the pharmacological activity of the compound being used. The determination of optimum dosages for a particular patient is well known to one of ordinary skill in the art.

In other embodiments, the compound of Formula I, Formula Ia, or Compound 1, can be administered to the patient concurrently with other cancer treatments. Such treatments include other cancer chemotherapeutics, hormone replacement therapy, radiation therapy, or immunotherapy, among others. The choice of other therapy will depend on a number of factors including the metabolic stability and length of action of the compound, the age, body weight, general health, sex, diet, mode and time of administration, rate of excretion, drug combination, the severity of the particular disease-states, and the host undergoing therapy.

In another aspect, the invention provides a method for detecting, diagnosing and treating *RET* fusions related disease such as *KIF5B-RET* fusion-positive NSCLC. We describe in more detail here methods for the detection and diagnosing of such disorders. Treating these disorders can be better accomplished with the administering of a therapeutically effective amount of a pharmaceutical composition comprising a Compound of Formula I or the malate salt of a Compound of Formula I or another pharmaceutically acceptable salt of a Compound of Formula I, including in a specific embodiment, the Compound of Formula I is Compound 1 or the malate salt of Compound 1 to a patient who has been identified or diagnosed as having a *RET* fusions related disease such as *KIF5B-RET* fusion-positive NSCLC. Descriptions of RET fusions follow.

### RET and KIF5B

The RET protein, the KIF5B protein, the KIF5B-RET fusion or sometimes simply called the "fusion protein and nucleic acids," including methods of detection, diagnosing, kits for detecting and diagnosing, methods of screening, methods of treating and preventing and various therapy for lung cancer patients, methods to measure the effectiveness of treatments and other pharmaceutical ingredients can be used in combination with the various treatments are described below.

The RET protein is a transmembrane receptor tyrosine kinase. The RET consists of extracellular region (which contains Cadherin-like domains), a trans-membrane domain and an intracellular region containing a tyrosine kinase domain. When the RET protein is dimerized by binding co-receptors and ligands, such as glial derived neurotrophic factor (GDNF), it is activated by auto-phosphorylation and then simulates downstream signaling pathways.

The RET protein may be derived from a mammal, such as a human. The human RET gene encoding the human RET protein is localized to chromosome 10 (10q11.2) and contains 19-21 exons depending on variants. The human RET protein may be encoded by a human RET gene. The C-terminal domain of RET protein may include an amino acid sequence encoded by a polynucleotide from 12^{th} exon to the last exon (for example, 20^{th} exon) of RET gene. The C-terminal domain of RET protein may include consecutive at least about 300 amino acids from the start position of 12^{th} exon. For example, the C-terminal domain of RET protein may include consecutive about 300 to about 450 amino acids, consecutive about 300 to about 420 amino acids, or consecutive about 300 to about 402 amino acids from the start position of 12^{th} exon (e.g., 713^{th} position) toward C-terminus of the RET protein (20 exons).

The KIF5B protein, which is also called as Kinesin-1 heavy chain, is a protein encoded by KIF5B gene. The KIF5B protein may be derived from a mammal, such as a human. The human KIF5B gene encoding the human KIF5B protein is localized to chromosome 10 (10q11.22) and contains 26 exons. The N-terminal domain of KIF5B protein may include an amino acid sequence encoded by a polynucleotide from the first exon to 16^{th} exon, or from the first exon to 15^{th} exon, or from the first exon to 23^{th} exon of KIF5B gene. The N-terminal domain of KIF5B protein may include consecutive at least about 329 amino acids from 1^{st} position (that is, at least amino acid sequence from 1^{st} to 329^{th} positions) of the KIF5B protein. The N-terminal domain of KIF5B protein may further include at least two coiled coil domain which starts from the amino acid of the 329^{th} position of the KIF5B protein. For example, the two coiled coil domain further included may have an amico acid sequence of 329^{th} to 638^{th} 20 positions of the KIF5B protein. The N-terminal domain of KIF5B protein may include consecutive about 329 to 900 amino acids, consecutive about 329 to 700 amino acids, consecutive about 329 to 650 amino acids, or consecutive about 329 to 638 amino acids from 1^{5t} position of the KIF5B protein.

The KIF5B-RET fusion is a RET-involved chromosomal rearrangement with the inversion or translocation on Chromosome 10. The fusion protein is detected and validated using various methods known to one skilled in the art and or as described herein. A composition of Formula I is then described for preventing or treating a lung cancer, comprising at least one inhibitor against the fusion protein, at least one inhibitor against the fusion gene encoding the fusion protein, at least one inhibitor against a RET coding gene, or a combination thereof, as an active ingredient.

In the fusion protein, the fusion, or fusion region may occur between various exons of the KIF5B gene and the RET gene. Many fusions are known to one skilled in the art. Examples of such fusions include the 20^{th}, or 16^{th} exon of KIF5B gene and 12^{th} exon of RET gene, which is called as a fusion point or breakpoint. Other RET and KIF5B fusion or breakpoints are known. The term "a fusion region" may refer to a polynucleotide fragment (about -30 nucleotides) or polypeptide (about -30 amino acids) fragment around the fusion point.

In other variants the fusion protein is described as any of the following: where the N-terminal domain of KIF5B protein consists essentially of consecutive at least about 329 amino acids from 1^{st} position of the KIF5B protein as described by the NCBI. The fusion protein wherein the N-terminal domain of KIF5B protein comprises at least two KIF5B coiled coil domain which starts from the amino acid of the 329^{th} position of the KIF5B protein. The fusion protein wherein the N-terminal domain 20 of KIF5B protein consists essentially of an amino acid sequence encoded by a polynucleotide from the first exon to 16^{th} exon, or from the first exon to 15^{th} exon, or from the first exon to 23^{th} exon of the polynucleotide as described by NCBI. Other known RET fusions on Chromosome 10 include KIF5B at exons 15, 16, 22, 23, 24 all with RET exon 12, and KIF5B at exon 24 with RET exon 11 and exon 8. CCDC6 at exon 1 and RET at exon 12; NCOA4 at exon 6 and RET at exon 12 and TRIM33 at exon 14 with RET at exon 12; all on chromosome 10 are known RET fusions. TRIM33 at exon 14 with RET at exon 12 are implicated in lung cancer and cancers of this type are known to respond well to cabozantinib administration. See, Cancer Discovery, Alexander Drilon, Lu Wang, Adnan Hasanovic, et al., Published OnlineFirst March 26, 2013; DOI: 10.1158/2159-8290.CD-13-0035, referring to American Association for Cancer Research, June 2013.

In one embodiment the fusion protein wherein the C-terminal domain of RET protein consists essentially of consecutive about 300 to 450 amino acids starting from an amino acid corresponding to the start position of 12^{th} exon of the fusion protein and then toward C-terminus of the RET protein.

As used herein, the exon number is numbered according to the exon number allocated by NCBI. The fusion protein KIF5B-RET may have any of the amino acid sequence identified as such by the NCBI. The nucleotide sequences of DNA molecules and the amino acid sequences of proteins encoded by the DNA molecules may be determined by an automated DNA sequencer or an automated peptide sequencer. The (nucleotide or amino acid) sequences determined by such automated sequencing means may include partial error compared with actual sequences. Generally the sequences determined by automated sequencing may have sequence identity of at least about 90%, at least 20 about 95%, at least about 99%, or at least about 99.9% compared with actual sequences. Therefore, the fusion protein, the fusion gene or the fusion region may have an amino acid sequence or a nucleotide sequence having sequence identity of at least about 90%, at least about 95%, at least about 99%, or at least about 99.9% compared with the sequences of identified as such by the NCBI.

The fusion protein, in some embodiments may consist of 638 N-terminal residues of KIF5B and 402 C-terminal residues of RET. The fusion gene has a protein tyrosine kinase domain together with a coiled-coil domain. The coiled-coil domain induces homo-dimerization which will activate the oncogenic protein tyrosine kinase domain by auto-phosphorylation. KIF5B is a microtubule-based motor protein, ubiquitously expressed due to its active promoter and involved in the transport of organelles in eukaryotic cells. Taken together, the KIF5B-RET fusion gene may be highly expressed and then dimerized after translation owing to KIF5B. Then, the dimerized RET protein tyrosine kinase domain may be stimulated abnormally, thus facilitating the stimulation of an oncogenic pathway.

Once a fusion is detected it will be known as a fusion gene of KIF5B-RET encoding the fusion protein of KIF5B-RET. A method of providing information for diagnosing a lung cancer, comprising the step of detecting, in a test sample obtained from a subject, a fusion as described herein. Diagnosis would compare a fusion gene encoding the fusion protein; and an overexpression of RET compared to a standard sample from an individual without a cancer, wherein when at least one element is selected and detected in the test sample, allowing the subject to be identified as any or all of the following: a cancer patient, a lung cancer patient, NSCLC lung cancer patient, a RET fusion related NSCLC patient, and/or a KIF5B-RET fusion related NSCLC patient.

The inversion of Chromosome 10 may be detected by using a polynucleotide (a probe) capable of hybridizing with (complementarily binding to) the inversion region in Chromosome 10 and/or a primer pair capable of detecting the inversion of Chromosome 10, for example, capable of producing a polynucleotide fragment having consecutive 100 to 200 nucleotides including the inversion region in Chromosome 10. The fusion protein, the fusion gene, and the fusion region are described herein. In a concrete embodiment, the fusion protein may also be detected by detecting the presence of the fusion protein or the fusion gene or mRNA corresponding to the fusion gene.

The presence of the fusion protein may be detected be a general assay that measures the interaction between the fusion protein and a material (e.g., an antibody or an aptamer) specifically binding to the fusion protein. The general assay may be immunochromatography, immunohistochemical staining, enzyme liked immunosorbent assay (ELISA), radioimmunoassay (RIA), enzyme immunoassay (EIA), florescence immunoassay (FIA), luminescence immunoassay (LIA), western blotting, FACS, and the like.

In addition, the presence of the fusion gene or the mRNA may be detected by a general assay such as PCR, FISH (fluorescent in situ hybridization), and the like, using a polynucleotide capable of hybridizing with (complementarily binding to) the fusion gene or the mRNA. FISH is described in more detail below. The fusion gene may be detected and/or validated by using the integration techniques of whole-transcriptome (RNA) and/or whole-genome DNA sequencing through massively parallel sequencing technologies. The polynucleotide capable of hybridizing with the fusion gene or the mRNA may be a siRNA, an oligonucleotide, DNA probe, or DNA primer, which can detect the fusion gene or the mRNA by a direct hybridization with the fused or truncated gene or transcript in the test sample.

A FISH assay can be performed using one or more probe sets, embodiments are provided such as: (1) A first probe set, which is a first probe set targeting a chromosomal site which contains the RET gene (first chromosomal site); it consists of a probe 1A labeled with a first fluorescent substance and a probe 1B labeled with a second fluorescent substance; probe 1A is complementary to the first region, which is the 5' region in the aforementioned first chromosomal site, probe 1B is complementary to the second region, which is present at a distance from the aforementioned first region and is the 3' region in the aforementioned first chromosomal site, and the breakpoint in the RET gene when the KIF5B-RET fusion gene is produced by a translocation between the KIF5B and RET genes is located in the 3' tail of the aforementioned first region, between the aforementioned first and second regions, or in the 5' tale of the aforementioned second region; (2) A second probe set, which is a second probe set targeting a chromosomal site which contains the KIF5B gene (second chromosomal site); it consists of a probe 2A labeled with a first fluorescent substance and a probe 2B labeled with a second fluorescent substance; probe 2A is complementary to the first region, which is the 5' region in the aforementioned second chromosomal site, probe 2B is complementary to the second region, which is present at a distance from the aforementioned first region and is the 3' region in the aforementioned second chromosomal site, and the breakpoint in the KIF5B gene when the KIF5B-RET fusion gene is produced by a translocation between the KIF5B and RET genes is located in the 3' tail of the aforementioned first region, between the aforementioned first and second regions, or in the 5' tale of the aforementioned second region; (3) a third probe set consisting of the aforementioned probe 2A and the aforementioned probe 1B; and (4) a fourth probe set consisting of a probe 4A which is complementary to the chromosomal site containing the RET gene (the third chromosomal site), and a probe 4B which is complementary to the chromosomal site containing the KIF5B gene (the fourth chromosomal site).

The length of the aforementioned first chromosomal site can be 0.5-2.0 Mb. The length of the aforementioned second chromosomal site can be 0.5-2.0 Mb. The length of the aforementioned third chromosomal site can be 0.5-2.0 Mb. The length of the aforementioned fourth chromosomal site can be 0.5-2.0 Mb.

A kit for detecting translocations between KIF5B and RET genes can include one or more probe sets. (1) A first probe set includes a probe 1A labeled with a first fluorescent substance and a probe 1B labeled with a second fluorescent substance; Probe 1A is complementary to the first region, which is the 5' region in the aforementioned first chromosomal site, probe 1B is complementary to the second region, which is present at a distance from the aforementioned first region and is the 3' region in the aforementioned first chromosomal site, and the breakpoint in the RET gene when the KIF5B-RET fusion gene is produced by a translocation between the KIF5B and RET genes is located in the 3' tail of the aforementioned first region, between the aforementioned first and second regions, or in the 5' tale of the aforementioned second region; (2) A second probe set, which is a second probe set targeting a chromosomal site which contains the KIF5B gene (second chromosomal site); it consists of a probe 2A labeled with a first fluorescent substance and a probe 2B labeled with a second fluorescent substance; probe 2A is complementary to the first region, which is the 5' region in the aforementioned second chromosomal site, probe 2B is complementary to the second region, which is present at a distance from the aforementioned first region and is the 3' region in the aforementioned second chromosomal site, and the breakpoint in the KIF5B gene when the KIF5B-RET fusion gene is produced by a translocation between the KIF5B and RET genes is located in the 3' tail of the aforementioned first region, between the aforementioned first and second regions, or in the 5' tale of the aforementioned second region; (3) A third probe set consisting of the aforementioned probe 2A and the aforementioned probe 1B; and a fourth probe set consisting of a probe 4A which is complementary to the chromosomal site containing the RET gene (the third chromosomal site) and a probe 4B which is complementary to the chromosomal site containing the KIF5B gene (the fourth chromosomal site).

A kit useful for identifying patients susceptible to RET-KIF5B translocation includes one or more elements selected from a group comprising an explanation of the use of the probes, a DNA contrast stain, a buffer for hybridization use, an encapsulant, and a control slide. The kit makes it possible to implement conveniently and efficiently the detection method of this invention. The kit can include as required elements (essential ingredients) the aforementioned first probe set, second probe set, third probe set or fourth probe set. Two or more types of probe sets can also be included in the kit. For example, the kit can incorporate first probe set and third probe set. Since the details for each probe set have been described above, they will not be repeated here.

"Detection of the presence or absence of a KIF5B-RET fusion polynucleotide" in can be performed directly using genome DNA that encodes the aforementioned fusion polypeptide or a transcript from that genome DNA, but it may also be performed indirectly using a translation product from that transcript (the aforementioned fusion polypeptide).

Because the genome DNA that encodes the fusion polypeptide is formed by inversion between the 10p11.2 and 10q11.2 regions, the phenomenon of this invention may be detected in the "detection of the presence or absence of a KIF5B-RET fusion polynucleotide." In this detection of inversion, for example, a split between a 5'-side region upstream from the kinase domain coding region of the RET gene and a 3'-side region downstream from that coding region of the RET gene may be detected, or a split between the region that encodes cadherin repeat and 5'-side region upstream from that coding region of the RET gene and the coding region of the transmembrane domain and 3'-side region downstream from that coding region of the RET gene may be detected, or a split between the coding region of part or all of the coiled coil domain and 5'-side region upstream from that coding region of the KIF5B gene and a 3'-side region downstream from the coding region of the coiled coil domain of the KIF5B gene may be detected.

Techniques known and available to the skilled practitioner may be used in the "detection of the presence or absence of a KIF5B-RET fusion polynucleotide" in the present invention. If "genome DNA that encodes the aforementioned fusion polypeptide" is the object, for example, *in situ* hybridization (ISH) using fluorescence or the like, genome PCR, direct sequencing, southern blotting, or genome microarray analysis may be used. If a "transcript from the aforementioned genome DNA" is the object, for example, RT-PCR, direct sequencing, northern blotting, dot blotting, or cDNA microarray analysis may be used.

The kit of this invention can also include other elements. Examples of these other elements are the specification for use of the probes, a DNA counterstain such as DAPI, a hybridization buffer, a wash buffer, solvents, mounting media, control slides, reaction vessels, and other equipment. Specifications for diagnostic purposes can also be included. Furthermore, specifications, etc. can also be included that show how the detection (positive identification) of KIF5B-RET translocation in chromosome samples from cancer patients should be set up in these patients using an RET kinase inhibitor. Moreover, a plan for determining the treatment course and an explanation of this plan can also be included.

Comparatively long probes (approximately 200 kb (probe 1A) to approximately 1,370 kb (probe 4B) are contemplated. Therefore, complementation between the probes and the target sequences does not need to be highly restrictive, to the extent that specific hybridization intended in this invention is achieved. An example of similarity between target sequences is at least 90%, preferably at least 95%, and more preferably at least 98%.

In the case where a first probe set and second probe set are used, the two fluorescence signals are separated and detected individually in chromosomal samples in which the translocation between the KIF5B gene and the RET gene occurred; in chromosomal samples in which the translocation did not occur, typically the two fluorescence signals are observed to be next to each other, or a signal (yellow) that is a combination of the two fluorescence signals is observed. Thus, the presence or absence of a translocation between the KIF5B gene and the RET gene is reflected in the pattern of the fluorescence signal. Consequently, a translocation between the KIF5B gene and the RET gene can be determined from the pattern of the fluorescence signal.

The judgment reached above is preferably and typically made based on a comparison of a result with a control (test sample). Here, the control is a chromosomal sample derived from a patient with non-small cell lung cancer or a chromosomal sample derived from a patient exhibiting precancerous lesions. In addition, chromosomal samples from a patient without precancerous lesions, chromosomal samples from patients who do not have cancer, or chromosomal samples taken from normal, healthy subjects can also be used as the control. Chromosomal samples derived from strains of cells can also be used as a control.

When the fusion gene is a fusion gene KIF5B-RET encoding the fusion protein of KIF5B-RET, the fusion gene KIF5B-RET may be detected by using a polynucleotide (a probe) capable of hybridizing with (complementarily binding to) the fusion region and/or a primer pair capable of producing a polynucleotide fragment having consecutive 100 to 200 nucleotides including the fusion region. In addition, the fusion protein KIF5B-RET may be detected using an antibody or aptamer specifically binding to the fusion region of the fusion protein KIF5B-RET.

In addition, the detection can be performed by a fusion assay which is a combination of chromogenic in situ hybridization (CISH) method and silver in situ hybridization (SISH) method. The "fusion point" in the present specification refers to a point where a portion derived from the respective genes of KIF5B is fused with a portion derived from the RET gene.

The term "capable of hybridizing with the fusion region (or the inversion region)" may refer to having a complementary sequence or a sequence having sequence identity of at least 90% with that of the fusion region (or the inversion region). Another embodiment provides a composition for diagnosing a cancer, including one or more selected from the group consisting of a polynucleotide capable of hybridizing with the fusion region, a primer pair capable of producing a polynucleotide fragment having consecutive 100 to 200 nucleotides including the fusion region. Also a polynucleotide capable of hybridizing with the inversion region in Chromosome 10, a primer pair capable of producing a polynucleotide fragment having consecutive 100 to 200 nucleotides including the inversion region of Chromosome 10, and an antibody or aptamer binding to the fusion region. Another embodiment provides a use of the fusion protein and/or the fusion gene for diagnosing a cancer. The patient may be any mammal, for example, a primate such as a human or monkey, a rodent such as a mouse or a rat, in particular a human. The test sample, suitable for use in any assays mentioned, may be a cell (e.g., a lung cell); a tissue (e.g., a lung tissue); body fluid (e.g., blood); circulating tumor DNA, circulating tumor cells. The samples may be collected in any manner known to one skilled in the art, including collection from the surgical biopsy of tumor, a core biopsy of tumor, a fine needle aspirate of tumor, pleural effusion, and other known methods of separating cells and tissues from patients. For example, a FISH assay (described herein) could be performed on circulating tumor cells.

The patient may be receiving treatments or have plans to be treated with a kinase inhibitor. The test sample may include a cell derived from a human cancer cell or an extract thereof.

Another embodiment provides a fusion gene encoding the fusion protein, where a gene encoding the N-terminal domain of the fusion partner positions at 5' end and a gene encoding the C-terminal domain of the RET protein positions at 3' end. In a concrete embodiment, when the fusion protein is the KIF5B-RET protein, the fusion gene may be represented as KIF5B-RET gene, where a gene encoding the N-terminal domain of KIF5B positions at 5' end and a gene encoding the C-terminal domain of the RET protein positions at 3' end.

Another embodiment provides an expression vector including the fusion gene and optionally transcription elements (e.g., a promoter and the like) operably linked to the fusion gene. Another embodiment provides a transformant cell transformed with the expression vector.

Biological specimens obtained in the course of treatment or diagnosis (biopsy samples, etc.) are often formalin fixed, but in this case, the use of *in situ* hybridization is advantageous because the DNA genome which is the detection object is stable under formalin fixing and because detection sensitivity is high.

In *in situ* hybridization, the genome DNA that encodes a KIF5B-RET fusion polypeptide in the biological specimen can be detected by hybridizing the polynucleotide of (a) or (b) stated below having a chain length of at least 15 bases:
(a) a polynucleotide that is at least one probe selected from the group made up of probes that hybridize to a polynucleotide that encodes the KIF5B protein and probes that hybridize to a polynucleotide that encodes the RET protein
(b) a polynucleotide that is a probe that hybridizes to a fusion site of a polynucleotide that encodes the KIF5B protein and a polynucleotide that encodes the RET protein.

However, the DNA sequence of a gene may mutate in the natural world (that is, non-artificially). Accordingly, such natural variants may also be the object of the present invention (similarly hereinafter).

The polynucleotide stated in (a) of the present invention may be any, provided that it can detect the presence of genome DNA that encodes the aforementioned KIF5B-RET fusion polypeptide in the biological specimen by hybridizing to a polynucleotide that encodes the KIF5B protein or a polynucleotide that encodes the RET protein, which are the target base sequences of that polynucleotide. It is preferably a polynucleotide stated in (a1) through (a4) below.
(a1) A combination of a polynucleotide that hybridizes to the coding region of part or all of the coiled coil domain and 5'-side region upstream from that coding region of the KIF5B gene (also referred to as "5' KIF5B probe 1" hereinafter) and a polynucleotide that hybridizes to the coding region of the kinase domain and 3'-side region downstream from that coding region of the RET gene (also referred to as "3' RET probe 1" hereinafter.)
(a2) A combination of a polynucleotide that hybridizes to the 5'-side region upstream from the coding region of the kinase domain of the RET gene (also referred to as "5' RET probe 1" hereinafter) and a polynucleotide that hybridizes to coding region of the kinase domain and 3'-side region downstream from that coding region of the RET gene (also referred to as "3' RET probe 1" hereinafter.)
(a3) A combination of a polynucleotide that hybridizes to the coding region of the cadherin repeat and 5'-side region upstream from that region of the RET gene (also referred to as "5' RET probe 2" hereinafter) and a polynucleotide that hybridizes to the coding region of the transmembrane domain and 3'-side region downstream from that coding region of the RET gene (also referred to as "3' RET probe 2" hereinafter.)
(a4) A combination of a polynucleotide that hybridizes to the coding region of part or all of the coiled coil domain and 5'-side region upstream from that coding region of the KIF5B gene (also referred to as "5' KIF5B probe 1" hereinafter) and a polynucleotide that hybridizes to the coding region of the coiled coil domain and 3'-side region downstream from that coding region of the KIF5B gene (also referred to as "3' KIF5B probe 1" hereinafter.)

The region (target base sequence) to which the polynucleotide of (a1) used in *in situ* hybridization hybridizes is preferably a region within 1,000,000 bases from the fusion site of the KIF5B gene and RET gene, for reasons of specificity to the target base sequence and detection sensitivity, and the region to which the polynucleotides of (a2) through (a4) used in *in situ* hybridization hybridize is preferably a region within 1,000,000 bases from the breakpoint in the KIF5B gene or RET gene, for the same reasons.

The polynucleotide stated in (a) or (b) above used in *in situ* hybridization is preferably a collection made up of a plurality of types of polypeptide that can cover all of the aforementioned target base sequences, for reasons of specificity to the target base sequence and detection sensitivity. In this case, the length of the polynucleotides that constitute the collection is at least 15 bases, and preferably 100 to 1000 bases.

The polynucleotide stated in (a) or (b) above used in *in situ* hybridization is preferably labeled by fluorescent dye or the like for detection. Examples of such a fluorescent dye include DEAC, FITC, R6G, TexRed and Cy5, but are not limited to these. Other than a fluorescent dye, the aforementioned polynucleotide may also be labeled with a dye (chromogen) such as DAB, or silver and the like based on enzymatic metal deposition.

In *in situ* hybridization, if 5' KIF1B probe 1 and 3' RET probe 1 are used, or if 5' RET probe 1 and 3' RET probe 1 are used, or if 5' RET probe 2 and 3' RET probe 2 are used, or if 5' KIF1B probe 1 and 3' KIF1B probe 1 are used, it is preferred that these probes are labeled with mutually different dyes. When *in situ* hybridization is performed using a combination of probes labeled with different dyes in this manner, when the signal produced by the label of 5' KIF1B probe 1 (for example, fluorescence) and the signal produced by the label of 3' RET probe 1 overlap, it can be determined that genome DNA that encodes a KIF5B-RET fusion polypeptide has been detected. On the other hand, when the signal produced by the label of 5' RET probe 1 and the signal produced by the label of 3' RET probe 1 are split, or the signal produced by the label of 5' RET probe 2 and the signal produced by the label of 3' RET probe 2 are split, or the signal produced by the label of 5' KIF1B probe 1 and the signal produced by the label of 3' KIF1B probe 1 are split, it can be determined that genome DNA that encodes a KIF5B-RET fusion polypeptide has been detected.

Polynucleotide labeling may be performed by a known technique. For example, a substrate base labeled with fluorescent dye or the like by nick translation or random priming may be integrated in a polynucleotide, thereby labeling that polynucleotide. In *in situ* hybridization, the conditions used when hybridizing the polynucleotide stated in (a) or (b) above and the aforementioned biological specimen may be varied depending on various factors such as the length of the relevant polynucleotide, but an example of high-stringency hybridization conditions is 0.2xSSC, 65°C, and an example of low-stringency hybridization conditions is 2.0xSSC, 50°C. Note that hybridization conditions of the same stringency as the aforementioned conditions can be achieved by a person skilled in the art appropriately selecting the various conditions such as salt concentration (dilution ratio of SSC) and temperature, as well as concentration of surfactant (NP-40, etc.), concentration of formamide, and pH.

Examples of methods for detecting genome DNA that encodes a KIF5B-RET fusion polypeptide using the polynucleotide stated in (a) or (b) above other than the aforementioned *in situ* hybridization are southern blotting, northern blotting and dot blotting. In these methods, the aforementioned fusion gene is detected by hybridizing the polynucleotide stated in (a) or (b) above to a membrane on which a nucleic acid extract obtained from the aforementioned biological specimen has been transcribed. When using the polynucleotide of (a), it can be determined that genome DNA that encodes a KIF5B-RET fusion polypeptide has been detected when a polypeptide that hybridizes to a polynucleotide that encodes the KIF5B protein and a polypeptide that hybridizes to a polynucleotide that encodes the RET protein recognize the same band developed on the membrane.

Genome microarray analysis and DNA microarray analysis are additional methods for detecting genome DNA that encodes a KIF5B-RET fusion polypeptide using the polynucleotide of (b) above. In these methods, an array of polynucleotides of (b) are fixed on a substrate, and the relevant genome DNA is detected by putting the biological specimen in contact with the polynucleotides on the array. In PCR or sequencing, the polynucleotide of (c) stated below may be used to specifically amplify part or all of a KIF5B-RET fusion polynucleotide, using DNA (genome DNA, cDNA) or RNA prepared from the biological specimen as a template. (c) A polynucleotide that is a pair of primers designed to sandwich a fusion site of a polynucleotide that encodes the KIF5B protein and a polynucleotide that encodes the RET protein. The "polynucleotide that is a pair of primers" is a primer set of which one primer hybridizes to a polynucleotide that encodes the KIF5B protein and the other primer hybridizes to a polynucleotide that encodes the RET protein in a base sequence such as the aforementioned fusion polynucleotide that serves as a target. The length of these polynucleotides is normally 15 to 100 bases, and preferably 17 to 30 bases.

From the viewpoints of precision and sensitivity of detection by PCR, the polynucleotide stated in (c) of the present invention is preferably a complementary sequence to the base sequence of the aforementioned fusion polynucleotide within 5000 bases from the fusion site of the polynucleotide that encodes the KIF5B protein and the polynucleotide that encodes the RET protein.

The "polynucleotide that is a pair of primers" can be appropriately designed by known techniques based on the base sequence of the KIF5B-RET fusion polynucleotide that serves as a target. Advantageous examples of the "polynucleotide that is a pair of primers" are primer sets made up of one primer selected from the group made up of KIF5B-RET-F1, KIF5B-int15-F1, KIF5B-int15-F2, KIF5B-ex16-F1, KIF5B-ex23-F1, KIF5B-ex24-F1, KIF5B-F-orf2438 and KIF5B-int15-F3.5, and one primer selected from the group made up of KIF5B-RET-R1, RET-int1 1-R3, RET-int7-R1, RET-int1 1-R0.5, RET-int1 1-R1, RET-int7-R2 and RET-R-orf2364. More preferably, it is KIF5B-RET-F1 and KIF5B-RET-R1, KIF5B-int15-F1 and KIF5B-RET-R1, KIF5B-int15-F2 and RET-int1 1-R3, KIF5B-ex16-F1 and KIF5B-RET-R1, KIF5B-ex23-F1 and KIF5B-RET-R1, or KIF5B-ex24-F1 primer and RET-int7-R1 primer.

The invention provides, methods of: identifying, assessing or detecting a KIF5B-RET fusion; methods of identifying, assessing, evaluating, and/or treating a subject having a cancer, e.g., a cancer having a KIF5B-RET fusion; isolated KIF5B-RET nucleic acid molecules, nucleic acid constructs, host cells containing the nucleic acid molecules; purified KIF5B-RET polypeptides and binding agents; detection reagents (e.g., probes, primers, antibodies, kits, capable, e.g., of specific detection of a KIF5B-RET nucleic acid or protein); screening assays for identifying molecules that interact with, e.g., inhibit, 5'KIF5B-3'RET fusions, e.g., novel kinase inhibitors; as well as assays and kits for evaluating, identifying, assessing and/or treating a subject having a cancer, e.g., a cancer having a KIF5B-RET fusion. The compositions and methods identified herein can be used, for example, to identify new KIF5B-RET inhibitors; to evaluate, identify or select subject, e.g., a patient, having a cancer; and to treat or prevent a cancer.

KIF5B-RET Nucleic Acid Molecules. In one aspect, the invention features a nucleic acid molecule (e.g., an isolated or purified) nucleic acid molecule that includes a fragment of a KIF5B gene and a fragment of a RET proto-oncogene. In one embodiment, the nucleic acid molecule includes a fusion, e.g., an in-frame fusion, of an exon of KIF5B (e.g., one more exons encoding a kinesin motor domain or a fragment thereof), and an exon of RET (e.g., one or more exons encoding a RET tyrosine kinase domain or a fragment thereof).

In an embodiment the 5'KIF5B-3'RET nucleic acid molecule comprises sufficient KIF5B and sufficient RET sequence such that the encoded 5'KIF5B-3'RET fusion has kinase activity, e.g., has elevated activity, e.g., kinase activity, as compared with wild type RET, e.g., in a cell of a cancer referred to herein. In an embodiment the encoded 5'KIF5B-3'RET fusion comprises at least 1, 2, 3, 4, 5, 6, 7, 9, 10, or 11 exons from KIF5B and at least 1, 2, 3, 4, 5, 6, 7, 9, or 10, RET exons. In one embodiment, the encoded 5'KIF5B-3'RET fusion polypeptide includes a kinesin motor domain, a coiled coil domain, or a functional fragment thereof, and a RET tyrosine kinase domain or a functional fragment thereof.

In one embodiment, the nucleic acid molecule includes a nucleotide sequence that has an in-frame fusion of exon 15 of KIF5B with exon 12 of RET (e.g., a sequence within an 11MB pericentric inversion on chromosome 10). In other embodiments, the nucleic acid molecules includes a nucleotide sequence in the region of 32,316,376-32,316,416 of chromosome 10 coupled to (e.g., juxtaposed to) nucleotides in the region of nucleotides 43,611,042-43,611,118 of chromosome 10. In another embodiment, the nucleic acid molecule includes a nucleotide sequence that includes a breakpoint. For example, the KIF5B-RET fusion can include an in-frame fusion of at least exon 15 of KIF5B or a fragment thereof (e.g., exons 1-15 of KIF5B or a fragment thereof) with at least exon 12 of RET or a fragment thereof (e.g., exons 12-20 of RET or a fragment thereof). In certain embodiments, the KIF5B-RET fusion is in a 5'-KIF5B to 3'-RET configuration. In one embodiment, the nucleic acid molecule includes the nucleotide sequence of exons 1-15 of the KIF5B gene, or a fragment thereof, or a sequence substantially identical thereto. In another embodiment, the nucleic acid molecule includes the nucleotide sequence of exons 12-20 of the RET gene, or a fragment thereof, or a sequence substantially identical thereto.

In other embodiments, the nucleic acid molecule includes a nucleotide sequence encoding a KIF5B-RET fusion polypeptide that includes a fragment of a KIF5B gene and a fragment of a RET proto-oncogene. In one embodiment, the nucleotide sequence encodes a KIF5B-RET fusion polypeptide that includes a kinesin motor domain or a functional fragment thereof, and a RET tyrosine kinase domain or a functional fragment thereof.

In another embodiment, the nucleic acid molecule includes a KIF5B-RET fusion that include a fusion junction between the RET transcript and the KIF5B transcript. In another embodiment, the nucleic acid molecule includes a fusion, e.g., an in-frame fusion, of at least exon 11 of RET or a fragment thereof (e.g., exons 1-11 of RET or a fragment thereof), and at least exon 16 or a fragment thereof (e.g., exons 16-25 of KIF5B or a fragment thereof). In certain embodiments, the KIF5B-RET fusion is in a 5'- RET to 3'- KIF5B configuration. In one embodiment, the nucleic acid molecule includes the nucleotides corresponding to exons 1-11 of a RET gene, or a fragment thereof, or a sequence substantially identical thereto.

In a related aspect, the invention features nucleic acid constructs that include the KIF5B-RET nucleic acid molecules described herein. In certain embodiments, the nucleic acid molecules are operatively linked to a native or a heterologous regulatory sequence. Also included are vectors and host cells that include the KIF5B-RET nucleic acid molecules described herein, e.g., vectors and host cells suitable for producing the nucleic acid molecules and polypeptides described herein.

In another aspect, the invention features nucleic acid molecules that reduces or inhibits the expression of a nucleic acid molecule that encodes a KIF5B-RET fusion described herein. Examples of such nucleic acid molecules include, for example, antisense molecules, ribozymes, RNAi, triple helix molecules that hybridize to a nucleic acid encoding KIF5B-RET, or a transcription regulatory region of KIF5B-RET, and blocks or reduces mRNA expression of KIF5B-RET.

The invention also features a nucleic acid molecule, e.g., nucleic acid fragment, suitable as probe, primer, bait or library member that includes, flanks, hybridizes to, which are useful for identifying, or are otherwise based on, the KIF5B-RET fusions described herein. In certain embodiments, the probe, primer or bait molecule is an oligonucleotide that allows capture, detection or isolation of a KIF5B-RET fusion nucleic acid molecule described herein. The oligonucleotide can comprise a nucleotide sequence substantially complementary to a fragment of the KIF5B-RET fusion nucleic acid molecules described herein. The sequence identity between the nucleic acid fragment, e.g., the oligonucleotide, and the target KIF5B-RET sequence need not be exact, so long as the sequences are sufficiently complementary to allow the capture, detection or isolation of the target sequence. In one embodiment, the nucleic acid fragment is a probe or primer that includes an oligonucleotide between about 5 and 25, e.g., between 10 and 20, or 10 and 15 nucleotides in length. In other embodiments, the nucleic acid fragment is a bait that includes an oligonucleotide between about 100 to 300 nucleotides, 130 to 230 nucleotides, 150 to 200 nucleotides, 200 to 350, 350 to 950, 300 to 600, 500- 1000, 750- 2000, nucleotides 00 nucleotides, in length and do not necessarily include the KIF5B-RET fusion nucleic acids.

In one embodiment, the nucleic acid fragment can be used to identify or capture, e.g., by hybridization, a KIF5B-RET fusion. For example, the nucleic acid fragment can be a probe, a primer, for use in identifying or capturing, e.g., by hybridization, a KIF5B-RET fusion described herein. In one embodiment, the nucleic acid fragment can be useful for identifying or capturing a KIF5B-RET breakpoint.. 1. In one embodiment, the nucleic acid fragment hybridizes to a nucleotide sequence within a chromosomal rearrangement that creates an in-frame fusion of exon 15 of KIF5B with exon 12 of RET (e.g., a sequence within an 11MB pericentric inversion on chromosome 10). In one embodiment, the nucleic acid fragment hybridizes to a nucleotide sequence in the region of 32,316,376-32,316,416 of chromosome 10 coupled to (e.g., juxtaposed to) nucleotides in the region of nucleotides 43,611,042-43,611,118 of chromosome 10.

The probes or primers described herein can be used, for example, for FISH detection or PCR amplification. In one exemplary embodiment where detection is based on PCR, amplification of the KIF5B-RET fusion junction can be performed using a primer or a primer pair, e.g., for amplifying a sequence flanking the KIF5B-RET fusion junctions described herein, e.g., the mutations or the junction of a chromosomal rearrangement described herein. In one embodiment, a pair of isolated oligonucleotide primers can amplify a region containing or adjacent to a position in the KIF5B-RET fusion. For example, forward primers can be designed to hybridize to a nucleotide sequence within KIF5B genomic or mRNA sequence.

The nucleic acid fragment can be detectably labeled with, e.g., a radiolabel, a fluorescent label, a bioluminescent label, a chemiluminescent label, an enzyme label, a binding pair label, or can include an affinity tag; a tag, or identifier (e.g., an adaptor, barcode or other sequence identifier).

A method of determining the presence of a KIF5B-RET fusion comprising: directly acquiring knowledge that a KIF5B-RET fusion nucleic acid molecule or polypeptide is present in a sample from a subject. The sample can be a sample comprised of fluid, cells, tissue, e.g., a tumor tissue, it can include a nucleic acid sample, a protein sample, a tumor biopsy or a circulating tumor cell or nucleic acid, it can be chosen from a lung cancer, including a NSCLC, a SCLC, a SCC, or a combination thereof and an adenocarcinoma or a melanoma.

The method of detecting the KIF5B-RET fusion in a nucleic acid molecule, and by the use of any of the following methods: nucleic acid hybridization assay, amplification-based assays, PCR-RFLP assay, real-time PCR, sequencing, screening analysis, FISH, spectral karyotyping or MFISH, comparative genomic hybridization), in situ hybridization, SSP, HPLC or mass-spectrometric genotyping.

The method detecting a KIF5B-RET fusion polypeptide is described including the method of contacting a protein sample with a reagent which specifically binds to a KIF5B-RET fusion polypeptide; and detecting the formation of a complex of the KIF5B-RET fusion polypeptide and the reagent. Methods of polypeptide detection include using a reagent labeled with a detectable group to facilitate detection of the bound and unbound reagent, wherein the reagent is an antibody molecule, wherein the level or activity the KIF5B-RET fusion is evaluated, wherein the KIF5B-RET fusion is detected prior to initiating, during, or after, a treatment in a subject, wherein the KIF5B-RET fusion is detected at the time of diagnosis with a cancer, wherein the KIF5B-RET fusion is detected at a predetermined interval, e.g., a first point in time and at least at a subsequent point in time.

Responses to treatment are also included, specifically where, responsive to a determination of the presence of the KIF5B-RET fusion, one or more of the following are used: (1) stratifying a patient population; (2) identifying or selecting the subject as likely or unlikely to respond to a treatment; (3) selecting a treatment option; and/or (4) prognosticating the time course of the disease in the subject.

An isolated or purified nucleic acid molecule that encodes a KIF5B-RET fusion or a breakpoint comprising fragment thereof. An isolated or purified nucleic KIF5B-RET nucleic acid molecule operatively linked to a native or a heterologous regulatory sequence. An isolated or purified vector comprising a nucleic acid molecule that encodes a KIF5B-RET fusion or a breakpoint comprising fragment thereof. A host cell comprising a vector. A nucleic acid molecule that specifically reduces or inhibits the expression of a nucleic acid molecule that encodes a KIF5B-RET fusion, which can be selected from an antisense molecule, ribozyme, siRNA, or triple helix molecule. An isolated or purified KIF5B-RET fusion polypeptide or breakpoint containing fragment thereof. The isolated or purified KIF5B-RET fusion polypeptide having a RET kinase activity, and/or a dimerizing or multimerizing activity. An isolated or purified antibody molecule that specifically binds a KIF5B-RET fusion polypeptide. The antibody molecule, wherein said antibody molecule is a monospecific antibody molecule to the KIF5B-RET fusion polypeptide.

Examples of the method for detecting the translation product of the KIF5B-RET polynucleotide in the present invention are immunostaining, western blotting, ELISA, flow cytometry, immunoprecipitation and antibody array analysis. In these methods, an antibody that binds to a KIF5B-RET fusion polypeptide is used. Examples of such an antibody are an antibody specific to a polypeptide containing a fusion site of the KIF5B protein and RET protein (also referred to as "fusion site-specific antibody" hereinafter), an antibody that binds to a polypeptide made up of a region on the C terminal side from the aforementioned fusion site of the RET protein (also referred to as "RET-C terminal antibody" hereinafter), and an antibody that binds to a polypeptide made up of a region on the N terminal side from the aforementioned fusion site of the KIF5B protein (also referred to as "KIF5B-N terminal antibody" hereinafter). Here, "fusion site-specific antibody" means an antibody that specifically binds to a polypeptide containing the aforementioned fusion site but does not bind to either wild-type (normal-type) KIF5B protein or wild-type (normal-type) RET protein.

A KIF5B-RET fusion polypeptide can be detected by the aforementioned fusion site-specific antibody or a combination of the aforementioned RET-C terminal antibody and KIF5B-N terminal antibody. However, since almost no expression of the RET protein, for example, is detected in normal lung cells, the presence of a KIF5B-RET fusion polypeptide in lung adenocarcinoma tissue can be detected even if the RET-C terminal antibody alone is used in immunostaining.

The "antibody that binds to a KIF5B-RET fusion polypeptide" can be prepared by a person skilled in the art selecting an appropriate known method. An example of such known methods is a method in which an immune animal is inoculated with the aforementioned polypeptide made up of a C terminal portion of the RET protein, a KIF5B-RET fusion polypeptide, the aforementioned polypeptide made up of an N terminal portion of the KIF5B protein, etc. thereby activating the immune system of the animal, and then the blood serum of the animal is recovered (polyclonal antibody), and methods for producing monoclonal antibodies such as the hybridoma method, recombinant DNA method and phage display method. If an antibody to which a labeled substance is bound is used, the target protein can be directly detected by detecting the label. The labeled substance is not particularly limited provided that it can bind to the antibody and can be detected. Examples include peroxidase, β-D-galactosidase, microperoxidase, horseradish peroxidase (HRP), fluorescein isothiocyanate (FITC), rhodamine isothiocyanate (RITC), alkali phosphatase, biotin, and radioactive substances. Furthermore, in addition to methods that directly detect a target protein using an antibody to which a labeled substance is bound, methods that indirectly detect the target protein using protein G, protein A or a secondary antibody to which a labeled substance is bound may also be used.

If the presence of a KIF5B-RET fusion polynucleotide is detected in a specimen isolated from a subject by the aforementioned methods, it is judged that efficacy of cancer treatment by a RET tyrosine kinase inhibitor such as Formula I, Ia or Compound 1 will be high in that patient, whereas if the presence of a KIF5B-RET fusion polynucleotide is not detected, it is judged that efficacy of cancer treatment by a RET tyrosine kinase inhibitor will be low in that patient.

As described above, any of the polynucleotides stated in (a) through (c) below having a chain length of at least 15 bases may be advantageously used in detecting the presence or absence of a KIF5B-RET fusion polynucleotide; (a) a polynucleotide that is at least one probe selected from the group made up of probes that hybridize to a polynucleotide that encodes the KIF5B protein and probes that hybridize to a polynucleotide that encodes the RET protein.; (b) a polynucleotide that is a probe that hybridizes to a fusion site of a polynucleotide that encodes the KIF5B protein and a polynucleotide that encodes the RET protein; (c) a polynucleotide that is a pair of primers designed to sandwich a fusion site of a polynucleotide that encodes the KIF5B protein and a polynucleotide that encodes the RET protein.

These polynucleotides have a base sequence complementary to the specific base sequence of the target gene. Here, "complementary" may mean not completely complementary, provided that it hybridizes. For example, these polypeptides have 80% or higher, preferably 90% or higher, more preferably 95% or higher, and most preferably 100% homology with the specified base sequence.

In the polynucleotides of (a) through (c), in part or all of DNA or RNA, nucleotides may be substituted with artificial nucleic acids such as PNA (polyamide nucleic acid, peptide nucleic acid), LNA (trademark, locked nucleic acid, bridged nucleic acid), ENA (trademark, 2'-O, 4'-C-ethylene-bridged nucleic acids), GNA (glycerol nucleic acid) and TNA (threose nucleic acid).

Further, as described above, an antibody that binds to a KIF5B-RET fusion polypeptide is advantageously used in detecting the translation product of a KIF5B-RET fusion polynucleotide. Accordingly, the present invention provides a drug for determining the effectiveness of cancer treatment by a RET tyrosine kinase inhibitor, comprising this antibody.

The detection method of a fusion gene of the present invention includes a step of detecting the presence of poly-nucleotides of the present specification in a sample obtained from a test subject. As the sample obtained from a test subject, substances collected from a test subject (samples isolated from a living body), specifically, any types of body fluid (preferably blood) collected, alveolar and bronchial washings, samples having undergone biopsy, and phlegm samples are used. Preferably, a biopsy sample or a phlegm sample from an affected area in the lung of a test subject is used. From the sample, genome DNA can be extracted and used. In addition, transcripts thereof (products produced as a result of transcription and translation of a genome; for example, mRNA, cDNA and proteins) can be used. Particularly, it is preferable to prepare and use mRNA or cDNA.

Genome DNA can be extracted by known methods, and the extraction can be easily performed using a commercially available DNA extraction kit.

The step of detection can be performed according to known gene analysis methods (for example, known methods that are commonly used as gene detection methods such as PCR, LCR (Ligase chain reaction), SDA (Strand displacement amplification), NASBA (Nucleic acid sequence-based amplification), ICAN (Isothermal and chimeric primer-initiated amplification of nucleic acids), LAMP (Loop-mediated isothermal amplification) method, TMA (Gen-Probe' s TMA system) method, in situ hybridization method, and microar-rays). For example, a hybridization technology in which a nucleic acid hybridized with a polynucleotide to be detected is used as a probe, a gene amplification technology in which DNA hybridized with a polynucleotide to be detected is used as a primer, or the like is used.

Specifically, the detection is performed using nucleic acids derived from a sample obtained from a test subject, for example, mRNA and the like. The amount of mRNA is measured by a method of gene amplification reaction by using primers that are designed so as to be able to specifically amplify the sequence of a polynucleotide to be detected. The primers used in the detection method of the present invention, or the primers included in the detection kit are not particularly limited as long as the primers can specifically amplify the sequence of a polynucleotide to be detected, and designed based on the base sequence of a polynucleotide to be detected. Primers used in the PCR amplification monitor method can be designed using primer design software (for example, Primer Express manufactured by PE Biosystems) and the like. In addition, since the larger the size of a PCR product is, the more the amplification efficiency worsens, it is appropriate for a sense primer and an antisense primer to be designed such that the size of amplification products obtained when mRNA or cDNA is amplified becomes 1 kb or less.

More specifically, a sense primer (5'-primer) is designed from a portion encoding KIF5B, and an antisense primer (3'-primer) is designed from a portion encoding RET. It is preferable to use the primer included in the detection kit of the present invention, and it is more preferable to use the primer that is most suitably included in the detection kit. In the PCR amplification monitor method, it is also possible to design multiplex PCR for detecting all fusion polynucleotides in a single reaction liquid, by mixing the above sense primers corresponding to respective genes. By the method suitable for each amplification technology, it is possible to confirm whether or not a target gene (whole gene or a specific portion thereof) has been amplified. For example, in the PCR method, PCR products are analyzed by agarose gel electrophoresis and subjected to ethidium bromide staining and the like, whereby it is possible to confirm whether or not amplified fragments having a target size have been obtained. When the amplified fragments having a target size have been obtained, this indicates that a polynucleotide to be detected is present in the sample obtained from a test subject. The presence of a polynucleotide to be detected can be detected in this manner.

The detection method of a fusion gene of the present invention preferably includes a step of detecting the presence of a specific polynucleotide in a sample obtained from a test subject by a gene amplification reaction and a step of detecting whether or not amplified fragments having a target size have been obtained.

The detection using a hybridization technology is performed using, for example, northern hybridization, dot blotting method, DNA microarray method, and RNA protection method. As probes used for hybridization, it is possible to use a probe which comprises sequences consisting of 16 bases respectively upstream and downstream of the fusion point as a center of a nucleic acid molecule consisting of at least 32 consecutive bases hybridizing with a polynucleotide to be detected or with a complementary strand thereof in a stringent condition (preferably in a more stringent condition), or comprises complementary strands thereof.

Hybridization can use either "stringent conditions" or "more stringent conditions" known to those skilled in the art. It is also possible to use a gene amplification technology such as RT-PCR. In the RT-PCR method, the PCR amplification monitor (real time PCR) method is performed during the process of gene amplification, whereby the presence of a polynucleotide to be detected can be more quantitatively analyzed. PCR amplification monitor methods can be used. Real time PCR is a known method, and can be simply performed using commercially available instruments and kits for this method.

The detection method of a fusion protein of some of the embodiments of the present invention includes a step of detecting the presence of a specific polypeptide in a sample obtained from a test subject, that is, a polypeptide encoded by a polynucleotide to be detected (hereinafter, called a polypeptide to be detected). Such a detection step can be performed by immunoassay method or enzyme activity assay method that is conducted by preparing a solubilized liquid derived from a sample obtained from a test subject (for example, a cancer tissue or cells obtained from a test subject) and combining a polypeptide to be detected contained in the liquid with an anti-KIF5B antibody and an anti-RET antibody. Preferably, it is possible to use techniques using a monoclonal antibody or a polyclonal antibody specific to a polypeptide to be detected, such as enzymatic immunoassay method, double antibodies sandwich ELISA method, fluorescence immunoassay method, radioimmunoassay method, and western blotting method.

When the polynucleotide to be detected or polypep-tide to be detected in the detection method of the present invention is detected from a sample obtained from a test subject, the test subject is a subject (patient) who has cancer with the polynucleotide positive and is to be provided with treatment using RET inhibitors.

The detection kit of the present invention comprises at least sense and antisense primers that are designed so as to be able to specifically amplify a polynucleotide to be detected in the detection method of the present invention. The sense and antisense primer set is a set of polynucleotides functioning as primers for amplifying a polynucleotide to be detected.

The primer set of the present invention in one embodiment comprises (1) a primer set which comprises a sense primer designed from a portion encoding KIF5B and an antisense primer designed from a portion encoding RET and is for detecting a fusion gene of KIF5B gene and RET gene, wherein the anti-sense primer consists of a nucleic acid molecule (preferably a nucleic acid molecule consisting of at least 16 bases) hybridizing with a "polynucleotide to be detected" under a stringent condition (preferably under a more stringent condition), and the sense primer consists of a nucleic acid molecule (preferably a nucleic acid molecule consisting of at least 16 bases) hybridizing with a complementary strand of the "polynucleotide to be detected" under a stringent condition (preferably under a more stringent condition).

The following primer sets (2) and (3) are included in the primer set as more specific variants of primer set (1).
(2) A primer set of a sense primer that consists of an oligonucleotide consisting of at least any 16 consecutive bases.
(3) A primer set of a sense primer that consists of an oligonucleotide consisting of at least any 16 consecutive bases.

In these primer sets (1) to (3), an interval between the positions where the sense primer and the anti-sense primer are selected is preferably 1 kb or less, or the size of an amplification product amplified by the sense primer and the antisense primer is preferably 1 kb or less.

In addition, the primer has a strand length consisting of 15 to 40 bases in general, preferably consisting of 16 to 24 bases, more preferably consisting of 18 to 24 bases, and particularly preferably consisting of 20 to 24 bases.

The primer set can be used for amplifying and detecting a polynucleotide to be detected. Moreover, though not particularly limited, the respective primers included in the primer set of the present invention can be prepared by, for example, chemical synthesis.

A method of screening an anticancer drug includes: contacting a sample compound to a cell expressing the fusion protein; and measuring the fusion protein expression level in the cell, wherein the fusion protein expression level in the cell treated with the sample compound is decreased compared with that before the treatment with the sample compound or that in a non-treated cell, the sample compound is determined as a candidate compound for the anticancer drug.

The method of screening an anticancer drug may further include the step of measuring the fusion protein expression level in the cell before the treatment of the sample compound. In this case the sample compound may be determined as a candidate compound for the anticancer drug when the fusion protein expression level after treatment of the sample compound is decreased compared with that before the treatment with the sample compound in the same cell. Alternatively, the method of screening an anticancer drug may include providing cells expressing the fusion protein, and contacting a sample compound to a part of the provided cells. In this case the sample compound may be determined as a candidate compound for the anticancer drug when the fusion protein expression level in the cell contacted with the sample compound is decreased compared with that in the cells which are not contacted with the sample compound.

The cell used in the screening method may be a cell derived from a cancer cell where the fusion gene or the fusion protein is expressed and/or activated, an extract of the cell, or a culture of the cell. The cancer cell may be a solid cancer cell, in particular a lung cancer, for example a non-small cell lung cancer such as a lung adenocarcinoma, as described above.

Still another embodiment provides a method of screening an anticancer drug against lung cancer including: treating a cell expressing the fusion protein with a sample compound; measuring the fusion protein expression level in the cell, wherein the fusion protein expression level in the cell treated with the sample compound is decreased compared with that before the treatment with the sample compound or that in a non-treated cell, the sample compound is determined as a candidate compound for the anticancer drug against lung cancer.

The KIF5B-RET fusion protein can be used as a marker for diagnosing a lung cancer or for treating or preventing or treating a lung cancer. The treatment or prevention of lung cancer comprises the step of administering a therapeutically effective amount of at least one inhibitor against the fusion protein, at least one inhibitor against the fusion gene encoding the fusion protein, at least one inhibitor against a RET coding gene, or a combination thereof, to a patient in need thereof. The inhibitor can be such as Formula I, Ia or Compound 1.

Another embodiment provides a method of preventing and/or treating a cancer, comprising administering a pharmaceutically (therapeutically) effective amount of at least one inhibitor against the fusion protein, at least one inhibitor against the fusion gene encoding the fusion protein, at least one inhibitor against a RET coding gene, or a combination thereof, to a patient in need thereof, any of which may be the compounds of Formula 1 and the other specific compounds disclosed herein. The method may further comprise the step of identifying the patient who needs the prevention and/or treatment of a cancer, prior to the step of administering such treatment. Another embodiment provides a composition for preventing and/or treating a cancer, comprising administering at least one inhibitor against the fusion protein, including Formula 1, either alone or with at least one inhibitor against the fusion gene encoding the fusion protein, at least one inhibitor against a RET coding gene, or a combination thereof. Another embodiment provides a use of an inhibitor against the fusion protein, an inhibitor against the fusion gene encoding the fusion protein, an inhibitor against a RET coding gene, or a combination thereof, for preventing and/or treating a cancer. The inhibitor can be such as Formula I, Ia or Compound 1.

In the present invention, the cancer may be a lung cancer, in particular a small cell lung cancer (SCLC) or a non-small cell lung cancer (NSCLC) such as a lung adenocarcinoma, a squamous cell lung carcinoma, or a large cell lung carcinoma.

The composition wherein the inhibitor against the fusion protein is at least one selected from the group consisting of an aptamer specifically binding to the fusion protein, an antibody 20 specifically binding to the fusion protein and the inhibitor against the fusion gene or the RET coding gene is at least one selected from the group consisting of siRNA, shRNA, miRNA, and an aptamer, which are capable of specifically binding to the fusion gene or the RET coding gene.

Any RET kinase inhibitor that inhibits the expression of RET and/or RET kinase activity (e.g., transcription, translation, or stability) may be used alone or in combination with the compound of Formula I, Ia, or 1.

The inhibiting agent may be RET-specific, or it may be non-specific (e.g., non-specific kinase inhibitors, multi-target inhibitors). Several RET kinase inhibitors have been developed, and their clinical applications are being investigated. Downstream of the RET kinase activity, there are kinases such as phosphatidylinositol 3 kinases (PI3K) and extracellular signal kinases 1/2 (ERK) (Wixted JH et al. J Biol Chem 2011), and STAT3 (Hwang JH et al. Mol Endocrinol 2003; 17: 1155-1166). A drug that inhibits such downstream signal transmission routes can also be used as an alternative to an RET kinase inhibitor or as an adjuvant, alone or in combination with the compounds of Formula 1.

In one mode, subjects judged to have a KIF5B-RET translocation are also judged as having an RET mutation at a site beyond the translocation site. An example of an RET mutation is an activating mutation. The RET activating mutation is an arbitrary mutation that causes an increase in activation in comparison with the wild type mutation. For example, an RET activating mutation may bring about permanent RET activation. There may even be a secondary RET activating mutation variant that is associated with or is due to the use of an RET inhibitor. Mutations that give rise to an increase in RET signal activity occur because of, for example, a kinase domain point mutation, deletion, insertion, duplication, or inversion, or combination of two or more of those, which give rise to an increase in the RET signal. For subjects who are judged to have both a KIF5B-RET translocation and an RET mutation, the decision can also be made to treat them with an RET kinase inhibitor. In addition, treatment/therapy may be carried out on them based on that decision.

As described above, the effectiveness of cancer treatment by a RET tyrosine kinase inhibitor is considered to be high in a patient in whom a KIF5B-RET fusion polynucleotide is detected by the method of the present invention. For this reason, cancer treatment can be efficiently performed by administering a RET tyrosine kinase inhibitor selectively to those cancer patients who possess the KIF5B gene and the RET gene. Accordingly, the present invention provides a method for treating cancer, comprising a step of administering a RET tyrosine kinase inhibitor which is Formula I, Ia or Compound 1 to a patient in whom the effectiveness of cancer treatment by that RET tyrosine kinase inhibitor was determined to be high by the aforementioned diagnostic method described herein.

In the present invention, a "specimen" is not only a biological specimen (for example, cells, tissue, organ, fluid (blood, lymph fluid, etc.), digestive fluid, sputum, alveolar/bronchial lavage fluid, urine, stools), but also includes nucleic acid extracts (genome DNA extract, mRNA extract, or cDNA preparation or cRNA preparation prepared from mRNA extract) or protein extracts obtained from these biological specimens. This specimen may also be one that has undergone formalin fixing treatment, alcohol fixing treatment, freezing treatment or paraffin embedding treatment.

Furthermore, the genome DNA, mRNA, cDNA or protein may be prepared by a person skilled in the art after selected a suitable known technique considering the type, state and so forth of the specimen.

In addition to the aforementioned substances (polynucleotides, antibodies) as active ingredients, the drug of the present invention may contain other pharmaceutically acceptable ingredients. Examples of such ingredients include buffering agents, emulsifiers, suspending agents, stabilizers, preservatives, physiological saline and so forth. As buffering agents, phosphates, citrates, acetates and so forth may be used. As emulsifiers, gum arabic, sodium alginate, tragacanth and so forth may be used. As suspending agents, glycerol monostearate, aluminum monostearate, methylcellulose, carboxymethylcellulose, hydroxymethylcellulose, sodium lauryl sulfate and so forth may be used. As stabilizers, propylene glycol, diethyl sulfite, ascorbic acid and so forth may be used. As preservatives, sodium azide, benzalkonium chloride, paraoxybenzoic acid, chlorobutanol and so forth may be used.

Furthermore, in addition to the preparation that contains polynucleotides and antibodies, preparations such as a substrate, a positive control (for example, KIF5B-RET fusion polynucleotides, KIF5B-RET fusion polypeptides, or cells that possess them, etc.) and a negative control necessary for detection of the label appended to the polynucleotides and antibodies, a counterstaining reagent (DAPI, etc.) used in *in situ* hybridization or the like, a molecule required in detecting the antibody (for example, a secondary antibody, protein G, protein A), and buffer solution used in dilution or washing of the antibody may be combined as a kit for use in the method of the present invention. This kit may include instructions for use of the kit. The present invention also provides the aforementioned kit for use in the method of the present invention.

The methods of detection described herein are particularly useful when a fusion protein is detected which consists essentially of N-terminal domain of a fusion partner and C-terminal domain of RET protein. The fusion protein may be KIF5B-RET fusion protein consisting essentially of N-terminal domain of KIF5B protein and C-terminal domain of RET protein. The method can be used for diagnosing a lung cancer, particularly non small cell lung cancer and includes: detecting at least one of aRET-involved chromosomal rearrangement including inversion or translocation in Chromosome 10; a fusion protein wherein RET protein is fused with other protein; a fusion gene encoding the fusion protein; and the overexpression of RET compared to a standard sample from an individual without a cancer. When one of the rearrangements described above selected from the above group is detected in the test sample, the individual for treatment of a RET inhibitor, such as a Formula I, Ia or Compound 1.

### Preparation of Compound 1

Preparation of N-(4-{[6,7-bis(methyloxy)quinolin-4-yl]oxy}phenyl)-N'-(4-fluorophenyl)cyclopropane-1,1-dicarboxamide and the (L)-malate salt thereof.

The synthetic route used for the preparation of N-(4-[6,7-bis(methyloxy)quinolin-4-yl]oxy}phenyl)-N'-(4-fluorophenyl)cyclopropane-1,1-dicarboxamide and the (L)-malate salt thereof is depicted in Scheme 1:

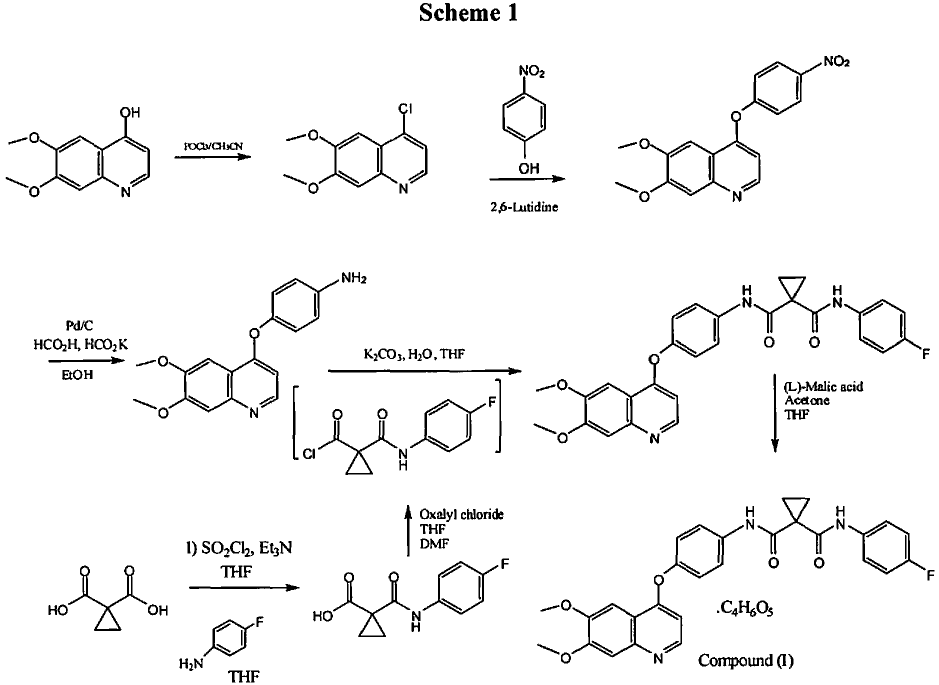

### Preparation of 4-Chloro-6,7-dimethoxy-quinoline

A reactor was charged sequentially with 6,7-dimethoxy-quinoline-4-ol (10.0 kg) and acetonitrile (64.0 L). The resulting mixture was heated to approximately 65 °C and phosphorus oxychloride (POCl₃, 50.0 kg) was added. After the addition of POCl₃, the temperature of the reaction mixture was raised to approximately 80 °C. The reaction was deemed complete (approximately 9.0 hours) when less than 2 percent of the starting material remained (in process high-performance liquid chromotography [HPLC] analysis). The reaction mixture was cooled to approximately 10 °C and then quenched into a chilled solution of dichloromethane (DCM, 238.0 kg), 30% NH₄OH (135.0 kg), and ice (440.0 kg). The resulting mixture was warmed to approximately 14 °C, and phases were separated. The organic phase was washed with water (40.0 kg) and concentrated by vacuum distillation to remove the solvent (approximately 190.0 kg). Methyl-t-butyl ether (MTBE, 50.0 kg) was added to the batch, and the mixture was cooled to approximately 10 °C, during which time the product crystallized out. The solids were recovered by centrifugation, washed with n heptane (20.0 kg), and dried at approximately 40 °C to afford the title compound (8.0 kg).

### Preparation of 6,7-Dimethyl-4-(4-nitro-phenoxy)-quinoline

A reactor was sequentially charged with 4-chloro-6,7-dimethoxy-quinoline (8.0 kg), 4 nitrophenol (7.0 kg), 4 dimethylaminopyridine (0.9 kg), and 2,6 lutidine (40.0 kg). The reactor contents were heated to approximately 147 °C. When the reaction was complete (less than 5 percent starting material remaining as determined by in process HPLC analysis, approximately 20 hours), the reactor contents were allowed to cool to approximately 25 °C. Methanol (26.0 kg) was added, followed by potassium carbonate (3.0 kg) dissolved in water (50.0 kg). The reactor contents were stirred for approximately 2 hours. The resulting solid precipitate was filtered, washed with water (67.0 kg), and dried at 25 °C for approximately 12 hours to afford the title compound (4.0 kg).

### Preparation of 4-(6,7 -Dimethoxy-quinoline-4-yloxy)-phenylamine

A solution containing potassium formate (5.0 kg), formic acid (3.0 kg), and water (16.0 kg) was added to a mixture of 6,7-dimethoxy-4-(4-nitro-phenoxy)-quinoline (4.0 kg), 10 percent palladium on carbon (50 percent water wet, 0.4 kg) in tetrahydrofuran (THF, 40.0 kg) that had been heated to approximately 60 °C. The addition was carried out such that the temperature of the reaction mixture remained approximately 60 °C. When the reaction was deemed complete as determined using in-process HPLC analysis (less than 2 percent starting material remaining, typically 1 5 hours), the reactor contents were filtered. The filtrate was concentrated by vacuum distillation at approximately 35 °C to half of its original volume, which resulted in the precipitation of the product. The product was recovered by filtration, washed with water (12.0 kg), and dried under vacuum at approximately 50 °C to afford the title compound (3.0 kg; 97 percent area under curve (AUC)).

### Preparation of 1-(4-Fluoro-phenylcarbamoyl)-cyclopropanecarboxylic acid

Triethylamine (8.0 kg) was added to a cooled (approximately 4 °C) solution of commercially available cyclopropane-1,1-dicarboxylic acid (2 1, 10.0 kg) in THF (63.0 kg) at a rate such that the batch temperature did not exceed 10 °C. The solution was stirred for approximately 30 minutes, and then thionyl chloride (9.0 kg) was added, keeping the batch temperature below 10 °C. When the addition was complete, a solution of 4-fluoroaniline (9.0 kg) in THF (25.0 kg) was added at a rate such that the batch temperature did not exceed 10 °C. The mixture was stirred for approximately 4 hours and then diluted with isopropyl acetate (87.0 kg). This solution was washed sequentially with aqueous sodium hydroxide (2.0 kg dissolved in 50.0 L of water), water (40.0 L), and aqueous sodium chloride (10.0 kg dissolved in 40.0 L of water). The organic solution was concentrated by vacuum distillation followed by the addition of heptane, which resulted in the precipitation of solid. The solid was recovered by centrifugation and then dried at approximately 35 °C under vacuum to afford the title compound. (10.0 kg).

### Preparation of 1-(4-Fluoro-phenylcarbamoyl)-cyclopropanecarbonyl chloride

Oxalyl chloride (1.0 kg) was added to a solution of 1-(4-fluoro-phenylcarbamoyl)-cyclopropanecarboxylic acid (2.0 kg) in a mixture of THF (11 kg) and N, N-dimethylformamide (DMF; 0.02 kg) at a rate such that the batch temperature did not exceed 30 °C. This solution was used in the next step without further processing.

### Preparation of N-(4-{[6,7-bis(methyloxy)quinolin-4-yl]oxy}phenyl)-N'-(4-fluorophenyl)cyclopropane-1,1-dicarboxamide

The solution from the previous step containing 1-(4-fluoro-phenylcarbamoyl)-cyclopropanecarbonyl chloride was added to a mixture of 4-(6,7-dimethoxy-quinoline-4-yloxy)-phenylamine (3.0 kg) and potassium carbonate (4.0 kg) in THF (27.0 kg) and water (13.0 kg) at a rate such that the batch temperature did not exceed 30 °C. When the reaction was complete (in typically 10 minutes), water (74.0 kg) was added. The mixture was stirred at 15-30 °C for approximately 10 hours, which resulted in the precipitation of the product. The product was recovered by filtration, washed with a pre-made solution of THF (11.0 kg) and water (24.0 kg), and dried at approximately 65 °C under vacuum for approximately 12 hours to afford the title compound (free base, 5.0 kg). ¹H NMR (400 MHz, d₆-DMSO): δ 10.2 (s, 1H), 10.05 (s, 1H), 8.4 (s, 1H), 7.8 (m, 2H), 7.65 (m, 2H), 7.5 (s, 1H), 7.35 (s, 1H), 7.25 (m, 2H), 7.15(m, 2H), 6.4 (s, 1H), 4.0 (d, 6H), 1.5 (s, 4H). LC/MS: M+H= 502.

### Preparation of N-(4-{[6,7-bis(methyloxy)quinolin-4-yl]oxy}phenyl)-N'-(4-fluorophenyl)cyclopropane-1,1-dicarboxamide, (L) malate salt

A solution of L-malic acid (2.0 kg) in water (2.0 kg) was added to a solution of Cyclopropane-1,1-dicarboxylic acid [4-(6,7-dimethoxy-quinoline-4-yloxy)-phenyl]-amide (4-fluoro-phenyl)-amide free base (1 5, 5.0 kg) in ethanol, maintaining a batch temperature of approximately 25 °C. Carbon (0.5 kg) and thiol silica (0.1 kg) were then added, and the resulting mixture was heated to approximately 78 °C, at which point water (6.0 kg) was added. The reaction mixture was then filtered, followed by the addition of isopropanol (38.0 kg), and was allowed to cool to approximately 25 °C. The product was recovered by filtration and washed with isopropanol (20.0 kg), and dried at approximately 65 °C to afford the title compound (5.0 kg).

### Alternative Preparation of N-(4-{[6,7-Bis(methyloxy)quinolin-4-yl]oxy}phenyl)-N'-(4-fluorophenyl)cyclopropane-1,1-dicarboxamide and the (L)-malate salt thereof.

An alternative synthetic route that can be used for the preparation of N-(4-{[6,7-bis(methyloxy)quinolin-4-yl]oxy}phenyl)-N'-(4-fluorophenyl)cyclopropane-1,1-dicarboxamide and the (L)-malate salt thereof is depicted in Scheme 2, as described in PCT/US2012/024591.

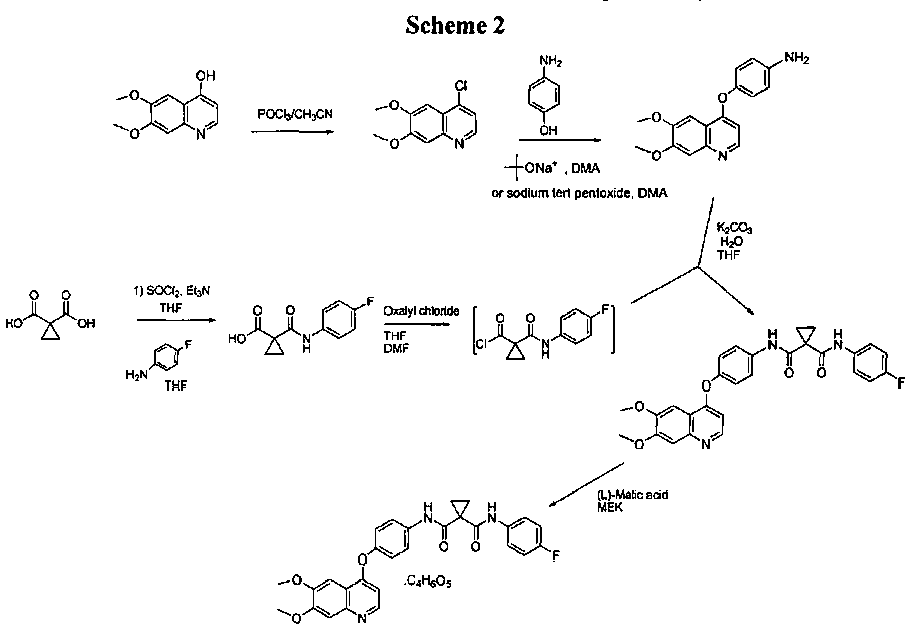

### Preparation of 4-Chloro-6,7-dimethoxy-quinoline

A reactor was charged sequentially with 6,7-dimethoxy-quinoline-4-ol (47.0 kg) and acetonitrile (318.8 kg). The resulting mixture was heated to approximately 60 °C and phosphorus oxychloride (POCl₃, 130.6 kg) was added. After the addition of POCl₃, the temperature of the reaction mixture was raised to approximately 77 °C. The reaction was deemed complete (approximately 13 hours) when less than 3% of the starting material remained (in-process high-performance liquid chromatography [HPLC] analysis). The reaction mixture was cooled to approximately 2-7 °C and then quenched into a chilled solution of dichloromethane (DCM, 482.8 kg), 26 percent NH₄OH (251.3 kg), and water (900 L). The resulting mixture was warmed to approximately 20-25 °C, and phases were separated. The organic phase was filtered through a bed of AW hyflo super-cel NF (Celite; 5.4 kg) and the filter bed was washed with DCM (118.9 kg). The combined organic phase was washed with brine (282.9 kg) and mixed with water (120 L). The phases were separated and the organic phase was concentrated by vacuum distillation with the removal of solvent (approximately 95 L residual volume). DCM (686.5 kg) was charged to the reactor containing organic phase and concentrated by vacuum distillation with the removal of solvent (approximately 90 L residual volume). Methyl t-butyl ether (MTBE, 226.0 kg) was then charged and the temperature of the mixture was adjusted to -20 to -25 °C and held for 2.5 hours resulting in solid precipitate which was then filtered and washed with n-heptane (92.0 kg), and dried on a filter at approximately 25 °C under nitrogen to afford the title compound. (35.6 kg).

### Preparation of 4-(6,7-Dimethoxy-quinoline-4-yloxy)-phenylamine

4-Aminophenol (24.4 kg) dissolved in N,N-dimethylacetamide (DMA, 184.3 kg) was charged to a reactor containing 4-chloro-6,7-dimethoxyquinoline (35.3 kg), sodium t-butoxide (21.4 kg) and DMA (167.2 kg) at 20-25 °C. This mixture was then heated to 100-105 °C for approximately 13 hours. After the reaction was deemed complete as determined using in-process HPLC analysis (less than 2 percent starting material remaining), the reactor contents were cooled at 15-20 °C and water (pre-cooled, 2-7 °C, 587 L) charged at a rate to maintain 15-30 °C temperature . The resulting solid precipitate was filtered, washed with a mixture of water (47 L) and DMA (89.1 kg) and finally with water (214 L). The filter cake was then dried at approximately 25 °C on filter to yield crude 4-(6, 7-dimethoxy-quinoline-4-yloxy)-phenylamine (59.4 kg wet, 41.6 kg dry calculated based on LOD). Crude 4-(6, 7-dimethoxy-quinoline-4-yloxy)-phenylamine was refluxed (approximately 75 °C) in a mixture of tetrahydrofuran (THF, 211.4 kg) and DMA (108.8 kg) for approximately 1hour and then cooled to 0-5 °C and aged for approximately 1 hour after which time the solid was filtered, washed with THF (147.6 kg) and dried on a filter under vacuum at approximately 25 °C to yield 4-(6,7-dimethoxy-quinoline-4-yloxy)-phenylamine (34.0 kg).

### Alternative Preparation of 4-(6, 7-Dimethoxy-quinoline-4-yloxy)-phenylamine

4-chloro-6,7-dimethoxyquinoline (34.8 kg) and 4-aminophenol (30.8 kg) and sodium tert pentoxide (1.8 equivalents) 88.7 kg, 35 weight percent in THF) were charged to a reactor, followed by N,N-dimethylacetamide (DMA, 293.3 kg). This mixture was then heated to 105-115 °C for approximately 9 hours. After the reaction was deemed complete as determined using in-process HPLC analysis (less than 2 percent starting material remaining), the reactor contents were cooled at 15-25 °C and water (315 kg) was added over a two hour period while maintaining the temperature between 20-30 °C. The reaction mixture was then agitated for an additional hour at 20-25 °C. The crude product was collected by filtration and washed with a mixture of 88kg water and 82.1 kg DMA, followed by 175 kg water. The product was dried on a filter drier for 53 hours. The LOD showed less than 1 percent w/w.

In an alternative procedure, 1.6 equivalents of sodium tert-pentoxide were used and the reaction temperature was increased from 110-120 °C. In addition, the cool down temperature was increased to 35-40 °C and the starting temperature of the water addition was adjusted to 35-40 °C, with an allowed exotherm to 45 °C.

### Preparation of 1-(4-Fluoro-phenylcarbamoyl)-cyclopropanecarboxylic acid

Triethylamine (19.5 kg) was added to a cooled (approximately 5 °C) solution of cyclopropane-1,1-dicarboxylic acid (24.7 kg) in THF (89.6 kg) at a rate such that the batch temperature did not exceed 5 °C. The solution was stirred for approximately 1.3 hours, and then thionyl chloride (23.1 kg) was added, keeping the batch temperature below 10 °C. When the addition was complete, the solution was stirred for approximately 4 hours keeping temperature below 10 °C. A solution of 4-fluoroaniline (18.0 kg) in THF (33.1 kg) was then added at a rate such that the batch temperature did not exceed 10 °C. The mixture was stirred for approximately 10 hours after which the reaction was deemed complete. The reaction mixture was then diluted with isopropyl acetate (218.1 kg). This solution was washed sequentially with aqueous sodium hydroxide (10.4 kg, 50 percent dissolved in 119 L of water) further diluted with water (415 L), then with water (100 L) and finally with aqueous sodium chloride (20.0 kg dissolved in 100 L of water). The organic solution was concentrated by vacuum distillation (100 L residual volume) below 40 °C followed by the addition of n-heptane (171.4 kg), which resulted in the precipitation of solid. The solid was recovered by filtration and washed with n-heptane (102.4 kg), resulting in wet, crude 1-(4-fluoro-phenylcarbamoyl)-cyclopropanecarboxylic acid (29.0 kg). The crude, 1-(4-fluoro-phenylcarbamoyl)-cyclopropanecarboxylic acid was dissolved in methanol (139.7 kg) at approximately 25 °C followed by the addition of water (320 L) resulting in slurry which was recovered by filtration, washed sequentially with water (20 L) and n-heptane (103.1 kg) and then dried on the filter at approximately 25 °C under nitrogen to afford the title compound (25.4 kg).

### Preparation of 1-(4-Fluoro-phenylcarbamoyl)-cyclopropanecarbonyl chloride

Oxalyl chloride (12.6 kg) was added to a solution of 1-(4-fluoro-phenylcarbamoyl)-cyclopropanecarboxylic acid (22.8 kg) in a mixture of THF (96.1 kg) and N, N-dimethylformamide (DMF; 0.23 kg) at a rate such that the batch temperature did not exceed 25 °C. This solution was used in the next step without further processing.

### Alternative Preparation of 1-(4-Fluoro-phenylcarbamoyl)-cyclopropanecarbonyl chloride

A reactor was charged with 1-(4-fluoro-phenylcarbamoyl)-cyclopropanecarboxylic acid (35 kg), 344 g DMF, and 175kg THF. The reaction mixture was adjusted to 12-17 °C and then to the reaction mixture was charged 19.9 kg of oxalyl chloride over a period of 1 hour. The reaction mixture was left stirring at 12-17 °C for 3 to 8 hours. This solution was used in the next step without further processing.

### Preparation of cyclopropane-1,1-dicarboxylic acid[4-(6,7-dimethoxy-quinoline-4-yloxy)-phenyl]-amide(4-fluoro-phenyl)-amide

The solution from the previous step containing 1-(4-fluoro-phenylcarbamoyl)-cyclopropanecarbonyl chloride was added to a mixture of compound 4-(6,7-dimethoxy-quinoline-4-yloxy)-phenylamine (23.5 kg) and potassium carbonate (31.9 kg) in THF (245.7 kg) and water (116 L) at a rate such that the batch temperature did not exceed 30 °C. When the reaction was complete (in approximately 20 minutes), water (653 L) was added. The mixture was stirred at 20-25 °C for approximately 10 hours, which resulted in the precipitation of the product. The product was recovered by filtration, washed with a pre-made solution of THF (68.6 kg) and water (256 L), and dried first on a filter under nitrogen at approximately 25 °C and then at approximately 45 °C under vacuum to afford the title compound (41.0 kg, 38.1 kg, calculated based on LOD).

### Alternative Preparation of cyclopropane-1,1-dicarboxylic acid[4-(6,7-dimethoxy-quinoline-4-yloxy)-phenyl]-amide(4-fluoro-phenyl)-amide

A reactor was charged with 4-(6,7-dimethoxy-quinoline-4-yloxy)-phenylamine (35.7 kg, 1 equivalent), followed by 412.9 kg THF. To the reaction mixture was charged a solution of 48.3 K₂CO₃ in 169 kg water. The acid chloride solution of described in the Alternative Preparation of 1-(4-Fluoro-phenylcarbamoyl)-cyclopropanecarbonyl chloride above was transferred to the reactor containing 4-(6,7-dimethoxy-quinoline-4-yloxy)-phenylamine while maintaining the temperature between 20-30 °C over a minimum of two hours. The reaction mixture was stirred at 20-25 °C for a minimum of three hours. The reaction temperature was then adjusted to 30-25 °C and the mixture was agitated. The agitation was stopped and the phases of the mixture were allowed to separate. The lower aqueous phase was removed and discarded. To the remaining upper organic phase was added 804 kg water. The reaction was left stirring at 15-25 °C for a minimum of 16 hours.

The product precipitated. The product was filtered and washed with a mixture of 179 kg water and 157.9 kg THF in two portions. The crude product was dried under a vacuum for at least two hours. The dried product was then taken up in 285.1 kg THF. The resulting suspension was transferred to reaction vessel and agitated until the suspension became a clear (dissolved) solution, which required heating to 30-35 °C for approximately 30 minutes. 456 kg water was then added to the solution, as well as 20 kg SDAG-1 ethanol (ethanol denatured with methanol over two hours. The mixture was agitated at 15-25 °C fir at least 16 hours. The product was filtered and washed with a mixture of 143 kg water and 126.7 THF in two portions. The product was dried at a maximum temperature set point of 40 °C.

In an alternative procedure, the reaction temperature during acid chloride formation was adjusted to 10-15 °C. The recrystallization temperature was changed from 15-25 °C to 45-50 °C for 1 hour and then cooled to 15-25 °C over 2 hours.

### Preparation of cyclopropane-1,1-dicarboxylic acid [4-(6,7-dimethoxy-quinoline-4-yloxy)-phenyl]-amide (4-fluoro-phenyl)-amide, malate salt

Cyclopropane-1,1-dicarboxylic acid [4-(6,7-dimethoxy-quinoline-4-yloxy)-phenyl]-amide (4-fluoro-phenyl)-amide (1-5; 13.3 kg), L-malic acid (4.96 kg), methyl ethyl ketone (MEK; 188.6 kg) and water (37.3 kg) were charged to a reactor and the mixture was heated to reflux (approximately 74 °C) for approximately 2 hours. The reactor temperature was reduced to 50 to 55 °C and the reactor contents were filtered. These sequential steps described above were repeated two more times starting with similar amounts of starting material (13.3 kg), L-Malic acid (4.96 kg), MEK (198.6 kg) and water (37.2 kg). The combined filtrate was azeotropically dried at atmospheric pressure using MEK (1133.2 kg) (approximate residual volume 711 L; KF ≤ 0.5 % w/w) at approximately 74 °C. The temperature of the reactor contents was reduced to 20 to 25 °C and held for approximately 4 hours resulting in solid precipitate which was filtered, washed with MEK (448 kg) and dried under vacuum at 50 °C to afford the title compound (45.5 kg).

### Alternative Preparation of cyclopropane-1,1-dicarboxylic acid[4-(6,7-dimethoxy-quinoline-4-yloxy)-phenyl]-amide(4-fluoro-phenyl)-amide, (L) malate salt

Cyclopropane-1,1-dicarboxylic acid [4-(6,7-dimethoxy-quinoline-4-yloxy)-phenyl]-amide (4-fluoro-phenyl)-amide (47.9 kg), L-malic acid (17.2), 658.2 kg methyl ethyl ketone, and 129.1 kg water (37.3 kg) were charged to a reactor and the mixture was heated 50-55 °C for approximately 1-3 hours, and then at 55-60 °C for an addition al 4-5 hours. The mixture was clarified by filtration through a 1 µm cartridge. The reactor temperature was adjusted to 20-25 °C and vacuum distilled with a vacuum at 150-200 mm Hg with a maximum jacket temperature of 55 °C to the volume range of 558-731 L.

The vacuum distillation was performed two more times with the charge of 380 kg and 380.2 kg methyl ethyl ketone, respectively. After the third distillation, the volume of the batch was adjusted to 18 v/w of cyclopropane-1,1-dicarboxylic acid [4-(6,7-dimethoxy-quinoline-4-yloxy)-phenyl]-amide (4-fluoro-phenyl)-amide by charging 159.9 kg methyl ethyl ketone to give a total volume of 880L. An addition al vacuum distillation was carried out by adjusting 245.7 methyl ethyl ketone. The reaction mixture was left with moderate agitation at 20-25 °C for at least 24 hours. The product was filtered and washed with 415.1 kg methyl ethyl ketone in three portions. The product was dried under a vacuum with the jacket temperature set point at 45 °C.

In an alternative procedure, the order of addition was changed so that a solution of 17.7 kg L-malic acid dissolved in 129.9 kg water was added to cyclopropane-1,1-dicarboxylic acid [4-(6,7-dimethoxy-quinoline-4-yloxy)-phenyl]-amide (4-fluoro-phenyl)-amide (48.7 kg) in methyl ethyl ketone (673.3 kg).

The invention includes the following specific embodiments.
Embodiment 1. A method for treating lung adenocarcinoma, comprising administering to a patient in need of such treatment a compound of Formula I: 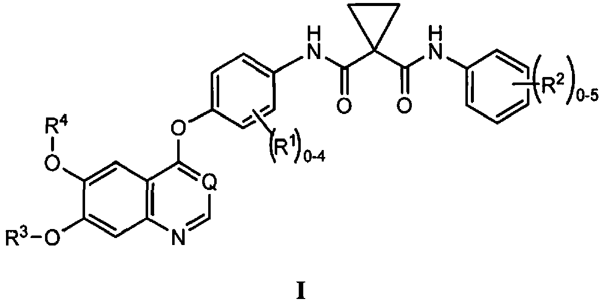 or a pharmaceutically acceptable salt thereof, wherein:
   R¹ is halo;
   R² is halo;
   R³ is (C₁-C₆)alkyl;
   R⁴ is (C₁-C₆)alkyl; and
   Q is CH or N.
Embodiment 2. The method of embodiment 1, wherein the lung adenocarcinoma is non-small cell lung cancer.
Embodiment 3. The method of embodiment 1, wherein the lung adenocarcinoma is *KIF5B-RET* fusion-positive non-small cell lung cancer.
Embodiment 4. The method of embodiment 1-3, wherein the dual MET and VEGF modulator is a compound of Formula Ia 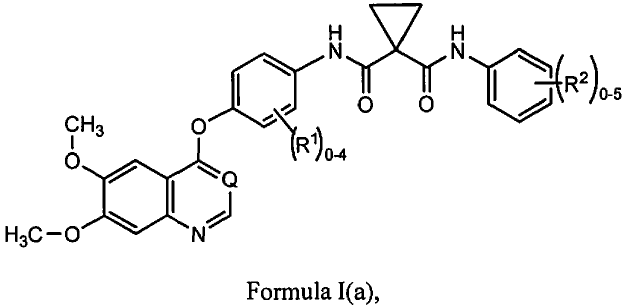 or a pharmaceutically acceptable salt thereof, wherein:
   R¹ is halo;
   R² is halo; and
   Q is CH or N.
Embodiment 5. The method of embodiment 1-4, wherein the compound of Formula I is Compounds 1: 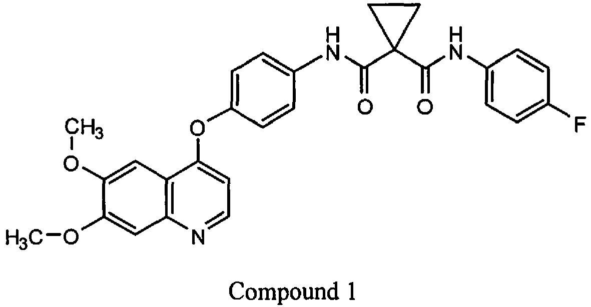 or a pharmaceutically acceptable salt thereof.
Embodiment 6. The method of embodiment 5, which is N-(4-{[6,7-bis(methyloxy)quinolin-4-yl]oxy}phenyl)-N'-(4-fluorophenyl)cyclopropane-1,1-dicarboxamide.
Embodiment 7. The method of embodiments 1-6, wherein the compound of Formula (I), Formula I(a) and Compound I is the (L)- or (D)-malate salt.
Embodiment 8. The method of embodiments 1-7, wherein the compound of Formula (I) is in the crystalline N-1 form or the N-2 form of the (L) malate salt and/or the (D) malate salt.
Embodiment 9. The method of embodiments 1-8 wherein the compound of Formula I, I(a), or Compound 1, or a pharmaceutically acceptable salt thereof, is administered as a pharmaceutical composition additionally comprising a pharmaceutically acceptable carrier, excipient, or diluent.
Embodiment 10. The method of embodiments 1-9 wherein the compound of Formula I is administered subsequent to another form of treatment.
Embodiment 11. The method of embodiments 1-9 wherein the compound of Formula I is administered post-cisplatin and/or gemcitabine treatment.
Embodiment 12. The method of embodiments 1-9 wherein the compound of Formula I is administered post-doectaxel treatment.
Embodiment 13. The method of embodiments 1-9 wherein the compound of Formula I is administered post-platinum (cisplatin or carboplatin) and/or paclitaxel, and/or gemcitabine, and/or docetaxel, and/or vinorelbine, and/or irinotecan, and and/or pemetrexed treatment.
Embodiment 14. A method for treating lung adenocarcinoma is *KIF5B-RET* fusion-positive non-small cell lung cancer in a patient in need of such treatment comprising administering a Compound 1 or a pharmaceutically acceptable salt thereof.
Embodiment 15. A method for inhibiting or reversing the progress of abnormal cell growth in a mammal, comprising administering Compound 1 or a pharmaceutically acceptable salt thereof, wherein the abnormal cell growth is cancer mediated by *KIF5B-RET.*
Embodiment 16. The method of embodiment 15, wherein the cancer is lung adenocarcinoma. 17. The method of embodiment 15, wherein the lung adenocarcinoma is non-small cell lung cancer.
Embodiment 18. The method of embodiment 15, wherein the lung adenocarcinoma is *KIF5B-RET* fusion-positive non-small cell lung cancer.
Embodiment 19. The method of embodiment 18, wherein Compound 1 or a pharmaceutically acceptable salt thereof is administered as a pharmaceutical composition comprising Compound 1 or a pharmaceutically acceptable salt thereof and at least one pharmaceutically acceptable carrier.
Embodiment 20. The method of embodiment 18, wherein Compound 1 or a pharmaceutically acceptable salt thereof is administered as a pharmaceutical composition comprising Compound 1 or a pharmaceutically acceptable salt thereof and at least one pharmaceutically acceptable carrier; wherein the pharmaceutical composition is administered daily for more than 3 months.
Embodiment 21. The method of embodiments 18, wherein Compound 1 or a pharmaceutically acceptable salt thereof is administered as a pharmaceutical composition comprising Compound 1 or a pharmaceutically acceptable salt thereof and at least one pharmaceutically acceptable carrier; wherein the pharmaceutical composition is administered at a dosage of 5, 10, 15, 20, 25, 30, 35, 40, 45, 50, 55, 65, 70, 75, 80, 85, 90, or 95 mg/day.
Embodiment 22. The method of embodiment 18, wherein the detection of the *KIF5B-RET* fusion-positive non-small cell lung cancer is made using a FISH, CISH or SISH assay.
Embodiment 23. The method of embodiment 18, wherein the detection of the *KIF5B-RET* fusion-positive non-small cell lung cancer is made using any form of genome PCR, direct sequencing, PCR sequencing, RT-PCR or similar assay.
Embodiment 24. A method of diagnosing and treating a patient wherein the patient has NSCLC tumor and the tumor is identified as *KIF5B-RET* fusion-positive NSCLC, and the treatment comprises the administration of any of the compounds of Formula I, including Compound 1, or a pharmaceutically acceptable salt thereof and at least one pharmaceutically acceptable carrier.
Embodiment 25. A method for treating a lung adenocarcinoma which is *KIF5B-RET* fusion positive non-small cell lung cancer in a patient in need of such treatment, comprising administering to the patient an effective amount of compound 1: 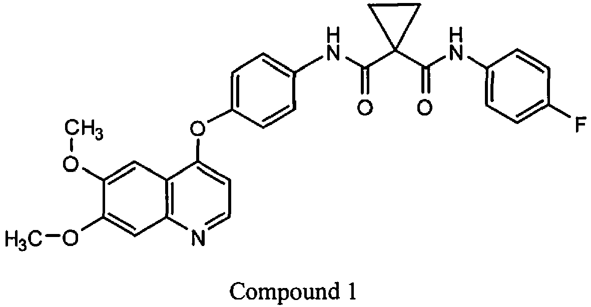 or a pharmaceutically acceptable salt thereof.
Embodiment 26. The method of embodiments 1-25, wherein the effective amount of a compound of Formula I, Ia, or 1 produces at least one therapeutic effect selected from the group consisting of reduction in size of a tumor, reduction in metastasis, complete remission, partial remission, stable disease, increase in overall response rate, or a pathologic complete response.

### Biological Example

### Compound 1 is a Potent Inhibitor of RET in Vitro

Compound 1 has been previously shown to be an ATP-competitive inhibitor of MET (IC₅₀ = 1.3 nmol/L) and VEGFR2 (IC₅₀ = 0.035 nmol/L) when profiled against a protein kinase panel of 270 human kinases. *See* Yakes, Mol Cancer Ther. 2011 Dec;10(12):2298-308. Compound 1 is also a potent inhibitor of RET with a biochemical IC₅₀ value of 5.2 nmol/L. RET-activating kinase domain mutations M918T and Y791F--known to be associated with hereditary and sporadic medullary thyroid carcinoma--were also inhibited by Compound 1 with IC₅₀ values of 27 and 1173 nmol/L, respectively. Moreover, Compound 1 was not active against the RET mutant V804L (IC₅₀ > 5000 nmol/L), which is known to render resistance to RET inhibitors. In cellular assays, Compound 1 inhibited RET autophosphorylation in TT cells, a calcitonin-expressing human medullary thyroid carcinoma cell line that harbors an activating C634W mutant of RET, with an IC₅₀ value of 85 nmol/L. The effect of Compound 1 on the growth of TT cells that were grown in 10% serum for 72 hours (3 days) was also invesitgated. Compound 1 treatment resulted in dose-dependent inhibition of proliferation with an IC₅₀ value of 94 nmol/L.

### Biological Example

### Compound 1 Inhibits Ligand-Independent Phosphorylation of RET In Vivo

TT-tumor bearing animals were administered single escalating doses of Compound 1 or water vehicle, and tumors were collected 4 h post dose. Levels of phosphorylated and total RET and were determined in pooled lysates by Western immunoblot analysis. In a separate study, mice bearing TT tumors were administered a single oral dose of cabozantinib (100 mg/kg) or water vehicle, and levels of phosphorylated and total RET, AKT, and ERK in tumor lysates were determined at the indicated time points post dose. Densitometric quantitation of the duration of inhibition of phosphorylation of RET versus plasma concentrations of cabozantinib. Representative Western blot images are shown.

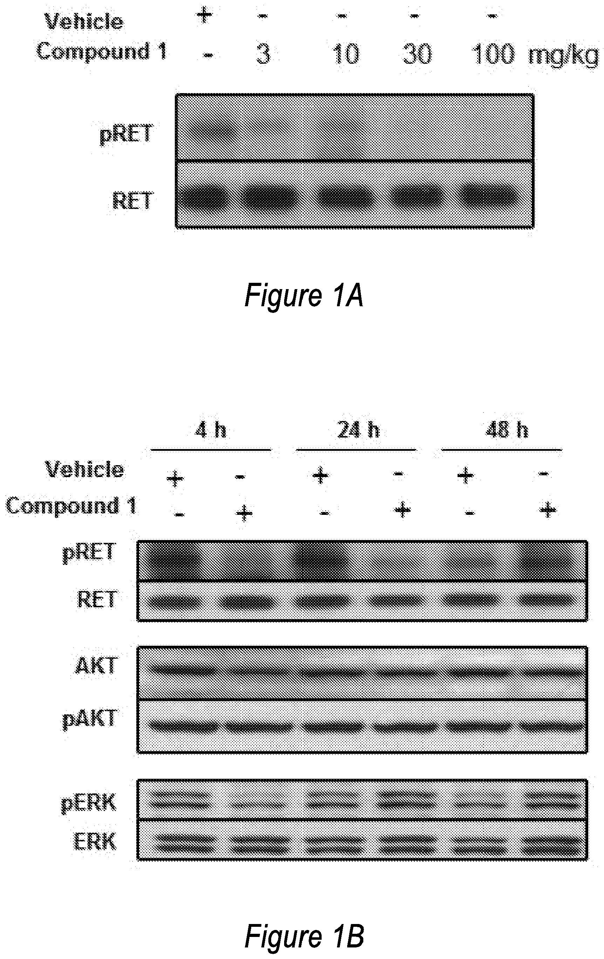

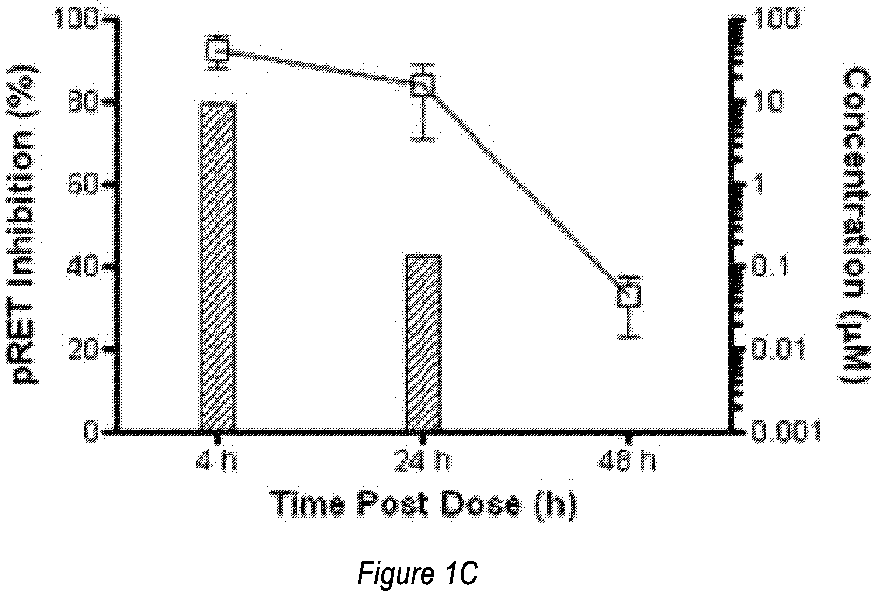

Single ascending oral dose administration of Compound 1 resulted in dose-dependent inhibition of phosphorylation of RET in the absence of reduced RET protein levels in TT xenograft tumors as depicted in Figure 1A. This result is consistent with data demonstrating the sensitivity of multiple medullary thyroid carcinoma cell lines to pharmacologic inhibitors selective for RET and RET knockdown by siRNA. Based on the dose-response relationship the predicted plasma concentration that results in 50% inhibition (IC₅₀) of phosphorylation of RET in this xenograft model is approximately 7 µmol/L. In a subsequent study, a single 100-mg/kg oral dose resulted in inhibition of phosphorylation of RET 4 to 24 hours post dose in TT xenograft tumors, as depicted in Figure IB. This effect was reversible as RET phosphorylation returned to basal levels by 48 hours after treatment as depicted in Figure 1C. In addition, Compound 1 reduced phosphorylation levels of AKT and ERK 4 to 24 hours post dose, which is consistent with inhibition of RET-mediated activation of the RAS/RAF/MAPK pathway. Plasma concentrations of Compound 1 associated with maximal and sustained inhibition of RET (15 µmol/L), AKT and ERK (42 µmol/L), respectively.

### Biological Example

### Compound 1 Inhibits TT Tumor Growth

The ability of Compound 1 to inhibit the growth of TT xenograft tumors was evaluated in *nu*/*nu* mice over a period of time corresponding to exponential tumor growth. *Nu*/*nu* mice bearing TT tumors were orally administered once daily water vehicle (□) or cabozantinib at 3 mg/kg (∇), 10 mg/kg (○), 30 mg/kg (◆), or 60 mg/kg (◇) for 21 days. Tumor weights were determined twice weekly. Data points represent the mean tumor weight (in milligrams) and SE for each treatment group. Circulating calcitonin levels were determined in serum preparations from whole blood collected after the final indicated doses (* indicates a significant, P < 0.05, reduction in circulating calcitonin when compared to serum samples from vehicle-treated control animals).

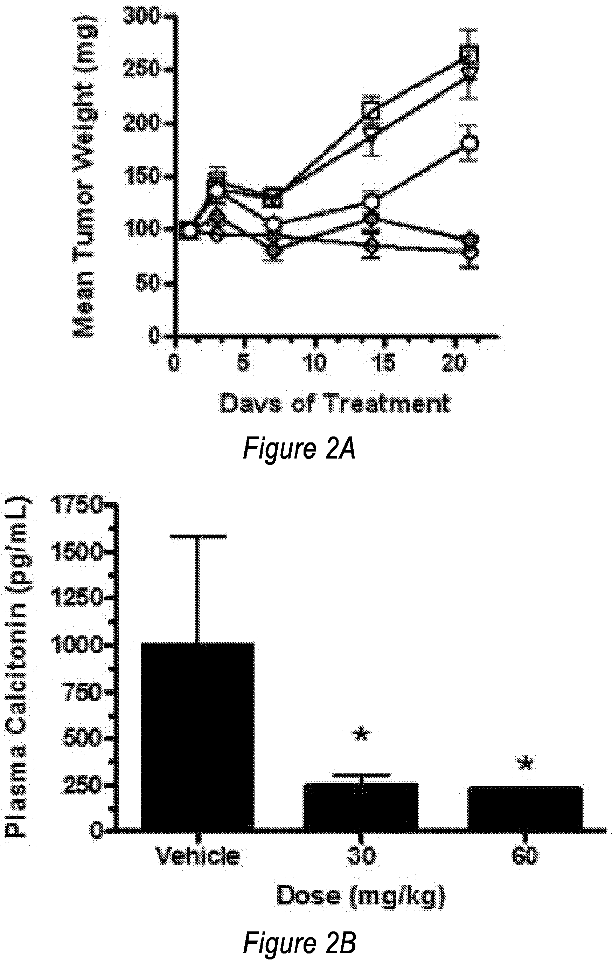

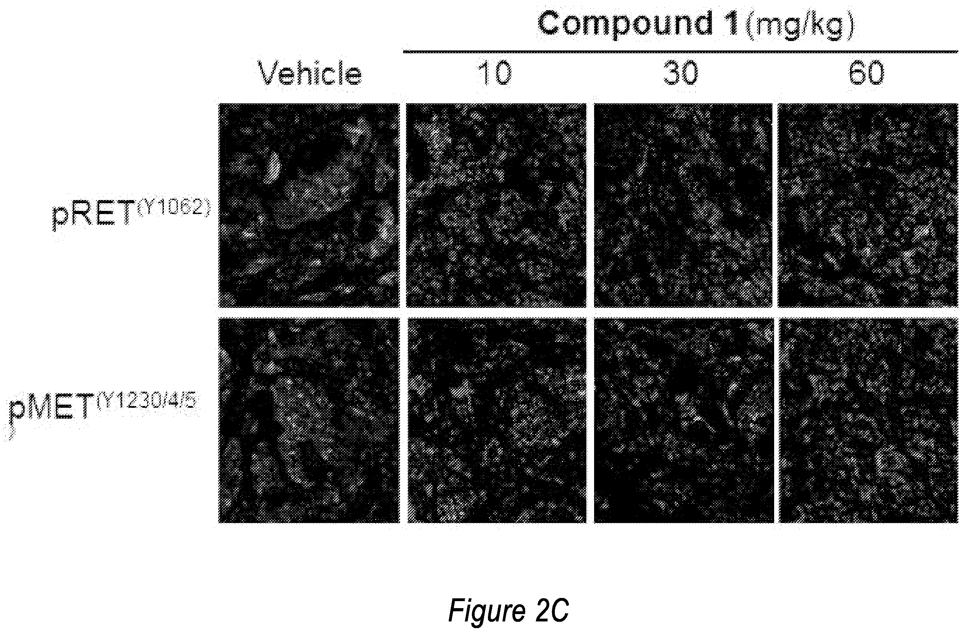

Compound 1 inhibits TT xenograft tumor growth that correlates with serum reductions in calcitonin, as depicted in Figure 2A with dose-dependent inhibition achieved for the 10- and 30-mg/kg doses. Furthermore, stable disease was observed at the 30- and 60-mg/kg doses that was associated with peak cyclical plasma concentrations of 3,000 to 45,000 nmol/L. Subchronic administration of Compound 1 was well tolerated as determined by stable body weights collected throughout the dosing period. Given that TT xenograft tumors are known to secrete high amounts of human calcitonin that correlates with tumor size, serum concentrations of circulating calcitonin were determined at the end of the dosing period. Serum from vehicle-treated control animals exhibited high levels of circulating calcitonin that was markedly reduced (75%; P < 0.005) at both the 30- and 60-mg/kg doses when compared to vehicle control animals, as depicted in Figure 2B. Moreover, this reduction in circulating plasma calcitonin correlated with TT tumor growth inhibition described above. Immunohistochemical analysis of tumors revealed significant and dose dependent decreases in levels of phosphorylated RET and MET as depicted in Figure 2C in the absence of reduced levels of total protein. Furthermore, Compound 1 treatment also resulted in dose dependent reductions in Ki67 and CD31 in viable tumor tissue indicating a negative impact on markers of cellular proliferation and vascularity as summarized in in Table 1.

**Table 1. Summary of Histochemical Analyses of TT Xenograft Tumors**

| Cabozantinib Dose (mg/kg) | RET^{(Y1062)} | | MET^{(Y1230/4/5)} | | CD31 | | Ki67 | |
|---|---|---|---|---|---|---|---|---|
| | Relative Area | Inhibition (%)^{a} | Relative Area | Inhibition (%)^{a} | Positive Cells (%) | Reduction (%)^{a} | Positive Cells (%) | Reduction (%)^{a} |
| Vehicle | 32.7 ± 2.6 | na | 27.4 ± 2.6 | na | 55.3 ± 6.9 | na | 26.6 ± 3.9 | na |
| 3 | 25.2 2.9 | 23 | 21.6 ± 2.7 | 21 | 35.9 ± 4.7 | 35 | 20.7 ± 2.6 | 22 |
| 10 | 17.4 1.9 | 47 | 17.2 ± 2.3 | 37 | 33.5 ± 4.9 | 39 | 19.4 ± 3.0 | 27 |
| 30 | 12.5 2.0 | 62 | 10.7 ± 1.5 | 61 | 26.4 ± 6.4 | 52 | 14.3 ± 3.9 | 46 |
| 60 | 9.7 2.1 | 70 | 8.2 ± 2.2 | 70 | 22.7 ± 8.6 | 59 | 8.1 ± 2.5 | 69 |
| ^{a}P<0.05 unless otherwise indicated | | | | | | | | |

### Case Study

A 51-year-old Japanese woman who was a former smoker presented in April 2009 for evaluation of a right pleural effusion. Computed tomography (CT) scans of the chest revealed a mass in the right middle lobe and right pleural effusion. Cytological examination of the pleural effusion revealed adenocarcinoma and *EGFR* was determined to be wild-type using high resolution melting analysis. A systemic workup showed no evidence of distant metastasis. There was also no tumor in either the neck or thyroid on the CT scans. The patient was diagnosed as having stage IIIB (cT4N0M0, 6th edition of the International System for Staging Lung cancer) adenocarcinoma of the lung. She was treated with 4 cycles of cisplatin and gemcitabine, and the primary tumor showed a partial response. Re-growth of primary tumor was, however, reported 8 months after the end of therapy. The patient subsequently received 13 cycles of docetaxel as second-line treatment and 2 cycles of an investigational drug (anti-HER2 [human epidermal growth factor receptor type2] antibody) as third-line treatment. In May 2011, she agreed to participate in the phase 1 study of Compound 1 monotherapy, and she received cabozantinib at a starting dose of 40 mg once a day. Yamamoto, N., Nokihara, H., Wakui, H., Yamada, Y., Frye, J., DeCillis, A., and Tamura, T. A phase 1 multiple ascending dose study of cabozantinib (XL184) monotherapy in Japanese patients with advanced solid tumors. Molecular Cancer Therapeutics. 2011 10 suppl 1 (abstr C26) and Nokihara, H., Yamamoto, N., Nakamichi, S., Wakui, H., Yamada, Y., Frye, J., Decillis, A., and Tamura, T. in Annals of Oncology, Vol. 23, Supplement 9, published September 17, 2012.

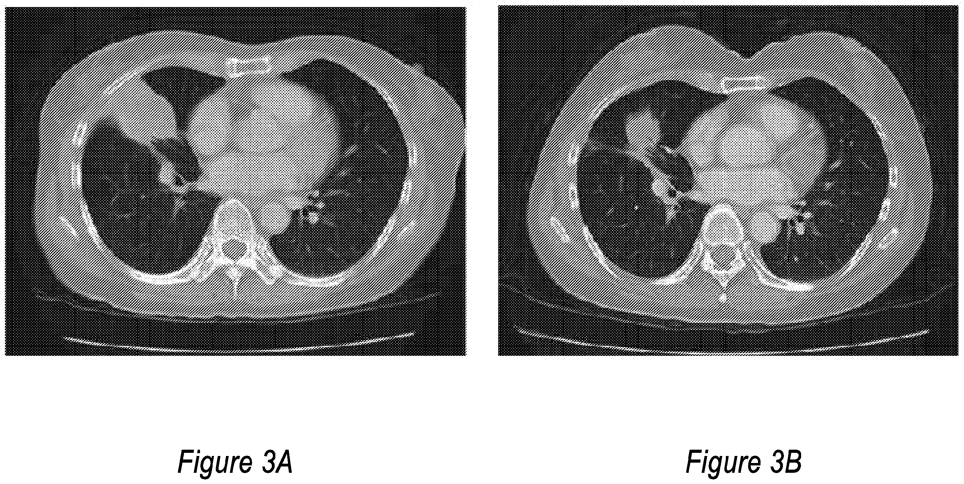

Chest CT scans at 9 weeks demonstrated partial response (40.1% tumor reduction) of her primary lung tumor (Figure 3), which was subsequently confirmed at 17 weeks. During the 10 cycles (months) of cabozantinib therapy, drug interruptions were employed due to grade 3 serum lipase elevations without clinical symptoms of pancreatitis or abnormal findings on abdominal ultrasonography. In February 2012, she terminated cabozantinib monotherapy due to progressive disease.

### Detection of KIF5B-RET fusion

The presence of *KIF5B-RET* fusion in this patient was evaluated retrospectively using pre- and post-treatment samples. Genomic DNA was extracted from pleural effusion cells at diagnosis as a pre-treatment sample, and genomic DNA and total RNA were extracted from pleural effusion cells at progression as a post-treatment sample. Genomic DNA was isolated using a QIAamp DNA Mini kit (Qiagen, Valencia, CA, USA). TRIzol (Invitrogen, Carlsbad, CA, USA) was used for the extraction of total RNA according to the manufacturer's instructions and quality was examined using a model 2100 bioanalyzer (Agilent Technologies, Santa Clara, CA, USA). The sample showed RNA Integrity Numbers > 6.0.

Total RNA (500 ng) was reverse-transcribed to cDNA using Superscript III Reverse Transcriptase (Invitrogen). cDNA (corresponding to 10 ng total RNA) or 10 ng genomic DNA was subjected to polymerase chain reaction (PCR) amplification using KAPA Taq DNA Polymerase (KAPA Biosystems, Woburn, MA, USA). The reactions were carried out in a thermal cycler under the following conditions: 40 cycles at 95°C for 15 sec, 60°C for 15 sec and 72°C for 1 min (for reverse transcriptase (RT) -PCR) or 3 min (for genomic PCR), with a final extension for 10 min at 72°C. The gene encoding glyceraldehyde-3-phosphate dehydrogenase (GAPDH) was amplified to estimate the efficiency of cDNA synthesis. The PCR products were directly sequenced in both directions using the BigDye Terminator kit and an ABI 3130x1 DNA Sequencer (Applied Biosystems, Foster City, CA, USA). This study was approved by the institutional review boards of the National Cancer Center in Tokyo, Japan. The PCR primers used in the present study are shown in Table 2.

**Table 2. PCR Primers**

| **Genomic PCR** | | | | | |
|---|---|---|---|---|---|
| No. | | Name | Location | Sequence | Use |
| 1 | | KIF5B-int15-F2.2 | KIF5B intron 15 | 5'-GGCATTTGACTTGGTGGTAGAT-3' | PCR |
| 2 | | KIF5B-RET-R1 | RET exon 12 | 5'-TCCAAATTCGCCTTCTCCTA-3' | PCR |
| 3 | | AD12-001Tseq-R1 | RET intron 11 | 5'-CCTGGGAACCCACAGTCAAG-3' | Sequencing |

| **RT-PCR** | | | | | |
|---|---|---|---|---|---|
| No. | | Name | Location | Sequence | Use |
| 3 | | KIF5B-867F | KIF5B exon 10 | 5'-ATTAGGTGGCAACTGTAGAACC-3' | PCR |
| 4 | | RET-2381R | KIF5B exon 12 | 5'-AGCCACAGATCAGGAAAAGA-3' | PCR |
| 5 | | KIF5B-RET-F1 | KIF5B exon 15 | 5'-AGGAAATGACCAACCACCAG-3' | Sequencing |
| 6 | | GAPDH-F | GAPDH exon 7 | 5'-CCAAGGTCATCCATGACAAC-3' | PCR |
| 7 | | GAPDH-R | GAPDH exon 9 | 5'-CACCCTGTTGCTGTAGCCA-3' | PCR |

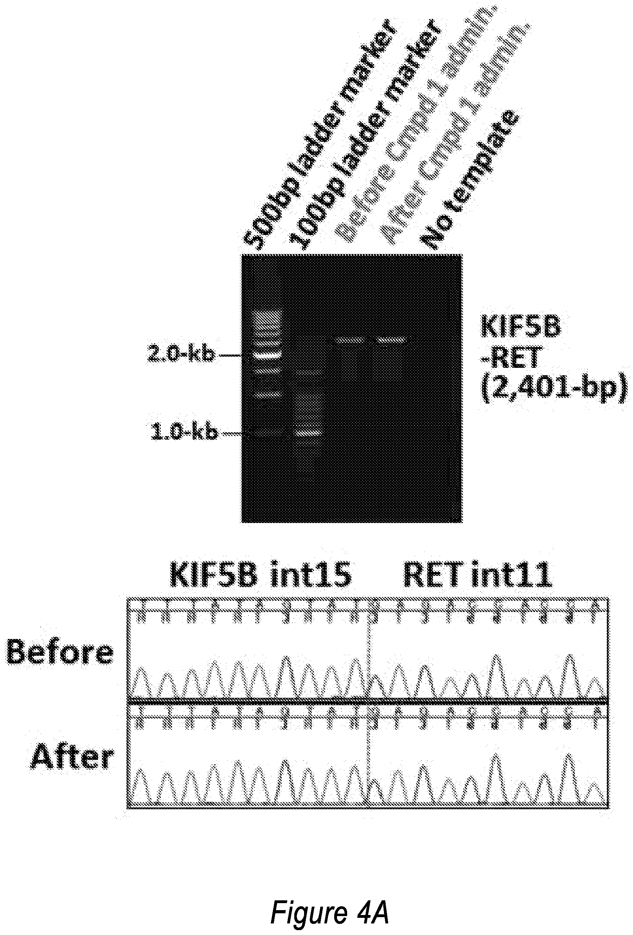

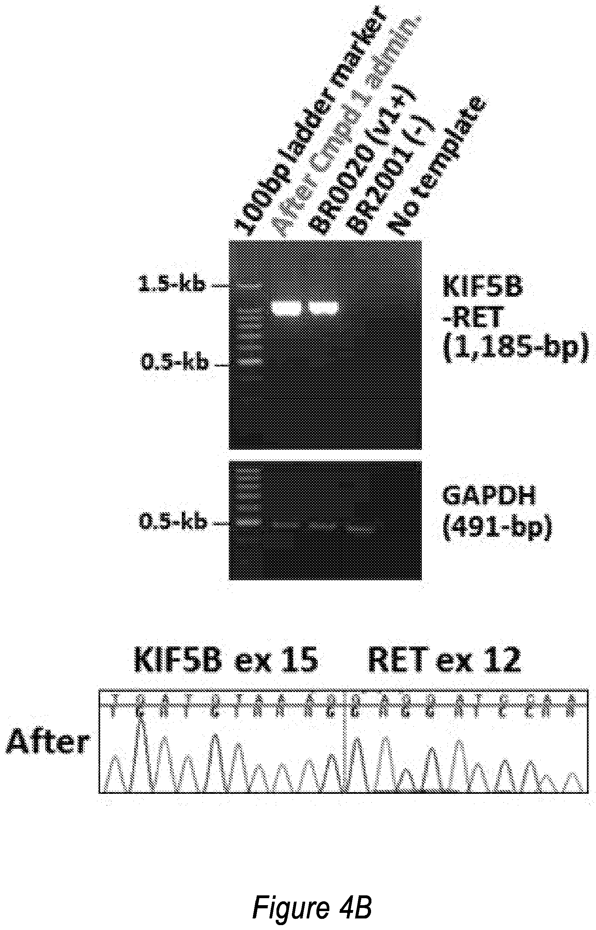

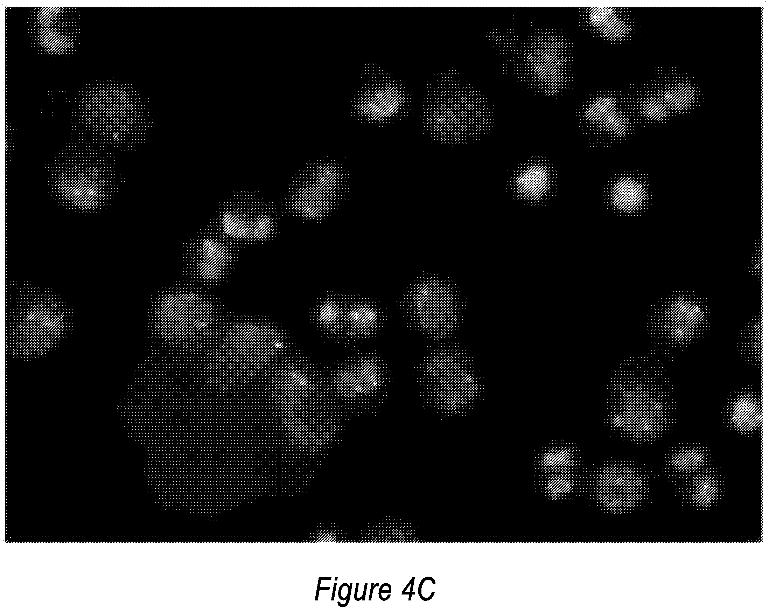

A fusion of the *KIF5B* (intron 15) and *RET* (intron 11) genes was detected in genomic DNAs in both pre- and post-treatment samples as depicted in Figure 4A, which shows KIF5B-RET genome PCR and Sanger sequencing from pre- and post-treatment tumor samples. Sanger sequencing of RT-PCR products verified the expression of variant 1 transcripts *(KIF5B* exon 15; *RET* exon 12), the most common type of *KIF5B-RET* fusion transcripts, in tumor cells, as depicted in Figure 4B, which shows KIF5B-RET RT-PCR and Sanger sequencing from post-treatment tumor sample. BR0020 (KIF5B-RET variant 1 fusion positive) and BR2001 (KIF5B-RET fusion negative) were used as positive and negative controls. *GAPDH* (glyceraldehyde-3-phosphate dehydrogenase) transcripts were amplified to estimate the quantity and quality of cDNAs. T, Ichikawa H, Totoki Y, Yasuda K, Hiramoto M, Nammo T, Sakamoto H, Tsuta K, Furuta K, Shimada Y, Iwakawa R, Ogiwara H, Oike T, Enari M, Schetter AJ, Okayama H, Haugen A, Skaug V, Chiku S, Yamanaka I, Arai Y, Watanabe S, Sekine I, Ogawa S, Harris CC, Tsuda H, Yoshida T, Yokota J, Shibata T. KIF5B-RET fusions in lung adenocarcinoma. Nat Med. 2012 Feb 12;18(3):375-7. Takeuchi K, Soda M, Togashi Y, Suzuki R, Sakata S, Hatano S, Asaka R, Hamanaka W, Ninomiya H, Uehara H, Lim Choi Y, Satoh Y, Okumura S, Nakagawa K, Mano H, Ishikawa Y. RET, ROS1 and ALK fusions in lung cancer. Nat Med. 2012 Feb 12;18(3):378-81. Lipson D, Capelletti M, Yelensky R, Otto G, Parker A, Jarosz M, Curran JA, Balasubramanian S, Bloom T, Brennan KW, Donahue A, Downing SR, Frampton GM, Garcia L, Juhn F, Mitchell KC, White E, White J, Zwirko Z, Peretz T, Nechushtan H, Soussan-Gutman L, Kim J, Sasaki H, Kim HR, Park SI, Ercan D, Sheehan CE, Ross JS, Cronin MT, Jänne PA, Stephens PJ. Identification of new ALK and RET gene fusions from colorectal and lung cancer biopsies. Nat Med. 2012 Feb 12;18(3):382-4. Cytological materials derived from the pre-treatment pleural effusion sample underwent fluorescent *in situ* hybridization (FISH) analysis using a break-apart *RET* probe set (Chromosome Science Labo Inc, Sapporo, Japan), which hybridizes with the neighboring 5' centromeric (RP11-379D20, labeled with Spectrum Green) and 3' telomeric (RP11-875A4, labeled with Spectrum Red) sequence of the *RET* gene as depicted in Figure 4C, which shows break-apart FISH at the *RET* locus. Tumor cells show split (5' green and 3' orange) signals in addition to fused signals (original magnification, 100×). A split signal defined by 5' and 3' probes observed at a distance > 1 times the signal size was observed in tumor cells. Thus, the tumor was judged to have a rearrangement of the *RET* gene, consistent with the PCR results above.

This is the first reported case in which a RET TKI has shown marked antitumor activity in a patient with *KIF5B-RET* fusion-positive NSCLC. To date, *in vitro* studies revealed that the growth and signaling properties mediated by KIF5B-RET were diminished after treatment with TKIs such as vandetanib, sunitinib or sorafenib. However, there has been no report that a patient with *KIF5B-RET* fusion-positive NSCLC responded to these drugs. Our report suggests that patients with advanced NSCLC harboring *KIF5B-RET* fusion may be exquisitely sensitive to therapeutic RET inhibition.

We have identified that approximately 2% of NSCLC patients harbor *KIF5B-RET* fusion. *KIF5B-RET* fusion-positive NSCLC comprises only a small subset of all lung cancers, however, lung cancer is a common disease and the number of lung cancer patients is increasing annually, so this subset translates into a considerable number of patients worldwide Therefore, the authors recommend development of a systematic screening method to identify *KIF5B-RET* fusion-positive NSCLC. The discovery of *EML4-ALK* rearrangements in NSCLC was published in 2007 and the US Food and Drug Administration approved crizotinib for this disease in 2011, followed by approval in Japan in 2012. Soda M, Choi YL, Enomoto M, Takada S, Yamashita Y, Ishikawa S, Fujiwara S, Watanabe H, Kurashina K, Hatanaka H, Bando M, Ohno S, Ishikawa Y, Aburatani H, Niki T, Sohara Y, Sugiyama Y, Mano H. Identification of the transforming EML4-ALK fusion gene in non-small-cell lung cancer. Nature. 2007 Aug 2;448(7153):561-6.This *KIF5B-RET-*positive patient had a marked clinical response to Compound 1, and this finding suggests that *KIF5B-RET* fusion is a driver oncogene in NSCLC and a promising therapeutic target.

Compound 1 is a potent inhibitor of TK against RET, a kinase that has been implicated in tumor pathobiology. For example, Yakes discloses that Compound 1 exhibits strong inhibition of RET, with an IC₅₀ of 5.2 ± 4.3 nMol/L. Yakes FM, Chen J, Tan J, Yamaguchi K, Shi Y, Yu P, Qian F, Chu F, Bentzien F, Cancilla B, Orf J, You A, Laird AD, Engst S, Lee L, Lesch J, Chou YC, Joly AH. Cabozantinib (XL184), a novel MET and VEGFR2 inhibitor, simultaneously suppresses metastasis, angiogenesis, and tumor growth. Mol Cancer Ther. 2011 Dec;10(12):2298-308. Activating mutations in *RET* play an important role in tumorigenesis in medullary thyroid cancer (MTC). Sennino B, Naylor RM, Tabruyn SP, You WK, Aftab DT McDonald DM. Reduction of tumor invasiveness and metastasis and prolongation of survival of RIP-Tag2 mice after inhibition of VEGFR plus c-Met by XL184. Mol Cancer Ther. 2009 8 suppl 1 (abstr A13). In a phase I dose-escalation study of cabozantinib, 25 (68%) of 37 patients with MTC had a confirmed partial response or stable disease for 6 months or longer. Kurzrock R, Sherman SI, Ball DW, Forastiere AA, Cohen RB, Mehra R, Pfister DG, Cohen EE, Janisch L, Nauling F, Hong DS, Ng CS, Ye L, Gagel RF, Frye J, Müller T, Ratain MJ, Salgia R. Activity of XL184 (Cabozantinib), an oral tyrosine kinase inhibitor, in patients with medullary thyroid cancer. J Clin Oncol. 2011 Jul 1;29(19):2660-6.In this study tumor regression was observed in patients with and without known *RET* mutations, suggesting some responses were caused by inhibition of targets other than RET, such as MET and/or VEGFR2, or as yet unknown aberrations in the RET pathway.

In summary, our NSCLC patient with *KIF5B-RET* fusion had a clinical response to cabozantinib, indicating that cabozantinib may be active in patients with advanced NSCLC harboring *KIF5B-RET* fusion. Urgent clinical evaluation of RET-TKIs against *KIF5B-RET* fusion-positive NSCLC is warranted.

## Claims

1. A compound for use in a method of treating lung adenocarcinoma in a patient, wherein the compound is the malate salt of Compound 1: 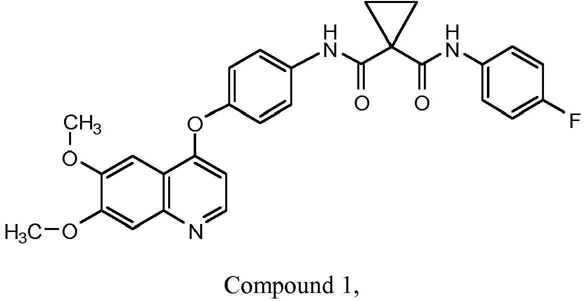 and wherein the lung adenocarcinoma is *KIF5B-RET* fusion-positive non-small cell lung cancer.

2. The compound for use of Claim 1, wherein the compound is the (L)- or (D)-malate salt of Compound 1.

3. The compound for use of Claim 1 or Claim 2, wherein the compound is administered post-docetaxel treatment.

4. A compound for use in a method of inhibiting or reversing the progress of cancer mediated by *KIF5B-RET* in a mammal, wherein the compound is the malate salt of Compound 1:

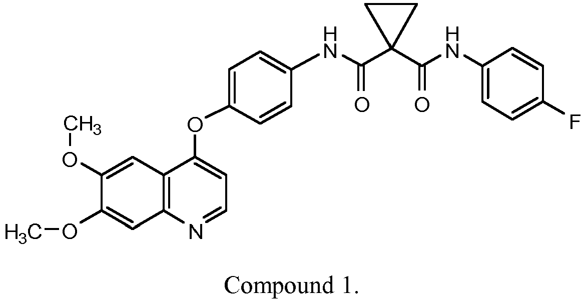

5. The compound for use of Claim 4, wherein the cancer mediated by *KIF5B-RET* is lung adenocarcinoma.

6. The compound for use of Claim 5, wherein the lung adenocarcinoma is non-small cell lung cancer.

7. The compound for use of Claim 6, wherein the lung adenocarcinoma is *KIF5B-RET* fusion-positive non-small cell lung cancer.

8. The compound for use of Claim 4, wherein the compound is administered as a pharmaceutical composition comprising the compound and at least one pharmaceutically acceptable carrier.

9. The compound for use of Claim 8, wherein the pharmaceutical composition is administered daily for more than 3 months.

10. The compound for use of Claim 4, wherein detection of the *KIF5B-RET* fusion-positive non-small cell lung cancer is made using a FISH, CISH or SISH assay.

11. The compound for use of Claim 4, wherein detection of the *KIF5B-RET* fusion-positive non-small cell lung cancer is made using any form of genome PCR, direct sequencing, PCR sequencing, RT-PCR or similar assay.

12. The compound for use of any one of Claims 1-11, wherein the compound produces at least one therapeutic effect selected from the group consisting of reduction in size of a tumor, reduction in metastasis, complete remission, partial remission, stable disease, increase in overall response rate, and a pathologic complete response.

13. A compound for use in a method of diagnosing and treating a patient wherein the patient has a NSCLC tumor and the tumor is identified as *KIF5B-RET* fusion-positive NSCLC, wherein the compound is the malate salt of Compound 1 as defined in Claim 1, and wherein the compound is administered with at least one pharmaceutically acceptable carrier.

14. The compound for use of any one of Claims 1-13, wherein Compound 1 is administered orally once daily as the malate salt as a capsule or tablet containing 20, 40, or 60 mg of Compound 1.

15. The compound for use of any one of Claims 1-14, wherein the compound is administered post- cisplatin and/or gemcitabine and/or docetaxel treatment.

## Patentansprüche

1. Eine Verbindung zur Verwendung in einem Verfahren zur Behandlung eines Lungenadenokarzinoms bei einem Patienten, wobei die Verbindung das Malatsalz von Verbindung 1 ist: 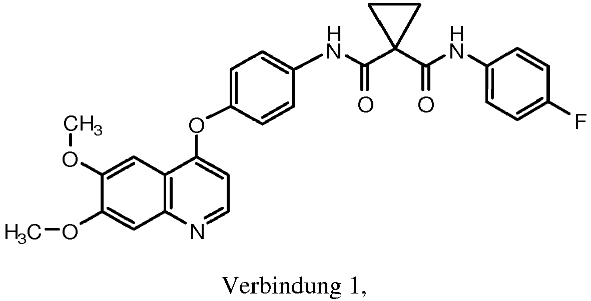 und wobei das Lungenadenokarzinom *KIF5B-RET*-fusionspositiver nichtkleinzelliger Lungenkrebs ist.

2. Verbindung zur Verwendung nach Anspruch 1, wobei die Verbindung das (L)- oder (D)-Malatsalz von Verbindung 1 ist.

3. Verbindung zur Verwendung nach Anspruch 1 oder Anspruch 2, wobei die Verbindung nach Behandlung mit Docetaxel verabreicht wird.

4. Eine Verbindung zur Verwendung in einem Verfahren zum Hemmen oder Umkehren des Forschritts von durch *KIF5B-RET* vermitteltem Krebs bei einem Säuger, wobei die Verbindung das Malatsalz von Verbindung 1 ist:

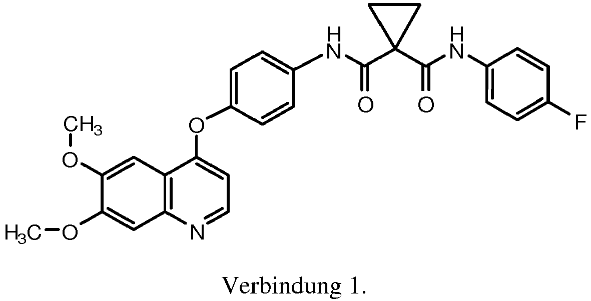

5. Verbindung zur Verwendung nach Anspruch 4, wobei der durch *KIF5B-RET* vermittelte Krebs Lungenadenokarzinom ist.

6. Verbindung zur Verwendung nach Anspruch 5, wobei das Lungenadenokarzinom nichtkleinzelliger Lungenkrebs ist.

7. Verbindung zur Verwendung nach Anspruch 6, wobei das Lungenadenokarzinom *KIF5B-RET-fusionspositiver* nichtkleinzelliger Lungenkrebs ist.

8. Verbindung zur Verwendung nach Anspruch 4, wobei die Verbindung als eine pharmazeutische Zusammensetzung verabreicht wird, die die Verbindung und mindestens einen pharmazeutisch akzeptablen Träger beinhaltet.

9. Verbindung zur Verwendung nach Anspruch 8, wobei die pharmazeutische Zusammensetzung mehr als 3 Monate lang täglich verabreicht wird.

10. Verbindung zur Verwendung nach Anspruch 4, wobei der Nachweis des *KIF5B-RET-*fusionspositiven nichtkleinzelligen Lungenkrebses unter Verwendung eines FISH-, CISH- oder SISH-Assays erfolgt.

11. Verbindung zur Verwendung nach Anspruch 4, wobei der Nachweis des *KIF5B-RET-*fusionspositiven nichtkleinzelligen Lungenkrebses unter Verwendung einer Form von Genom-PCR, direkter Sequenzierung, PCR-Sequenzierung, RT-PCR oder eines ähnlichen Assay erfolgt.

12. Verbindung zur Verwendung nach einem der Ansprüche 1-11, wobei die Verbindung mindestens eine therapeutische Wirkung hervorruft, ausgewählt aus der Gruppe, bestehend aus Reduktion der Größe eines Tumors, Reduktion der Metastasierung, komplette Remission, Teilremission, stabile Erkrankung, Steigerung der Gesamtansprechrate und eine komplettes pathologisches Ansprechen.

13. Eine Verbindung zur Verwendung in einem Verfahren zum Diagnostizieren und Behandeln eines Patienten, wobei der Patient einen NSCLC-Tumor hat und der Tumor als *KIF5B-RET-fusionspositiver* NSCLC identifiziert ist, wobei die Verbindung das Malatsalz von Verbindung 1 wie in Anspruch 1 definiert ist und wobei die Verbindung mit mindestens einem pharmazeutisch akzeptablen Träger verabreicht wird.

14. Verbindung zur Verwendung nach einem der Ansprüche 1-13, wobei Verbindung 1 einmal täglich oral als das Malatsalz als eine Kapsel oder Tablette, die 20, 40 oder 60 mg von Verbindung 1 enthält, verabreicht wird.

15. Verbindung zur Verwendung nach einem der Ansprüche 1-14, wobei die Verbindung nach Behandlung mit Cisplatin und/oder Gemcitabin und/oder Docetaxel verabreicht wird.

## Revendications

1. Composé destiné à une utilisation dans une méthode de traitement d'un adénocarcinome pulmonaire chez un patient, le composé étant le sel malate du Composé 1 : 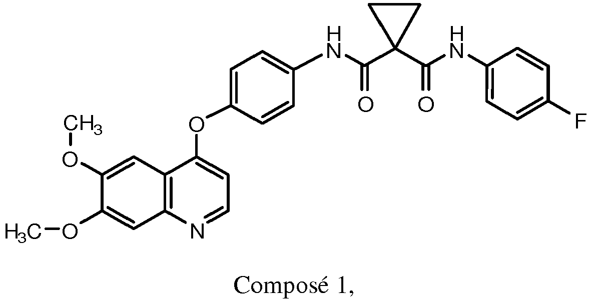 et dans lequel l'adénocarcinome pulmonaire est un cancer du poumon non à petites cellules positif pour le gène de fusion *KIF5B-RET.*

2. Composé destiné à une utilisation selon la revendication 1, le composé étant le sel L-malate ou D-malate du Composé 1.

3. Composé destiné à une utilisation selon la revendication 1 ou la revendication 2, le composé étant administré après un traitement par docétaxel.

4. Composé destiné à une utilisation dans une méthode d'inhibition ou d'inversion de la progression d'un cancer médié par *KIF5B-RET* chez un mammifère, le composé étant le sel malate du Composé 1 :

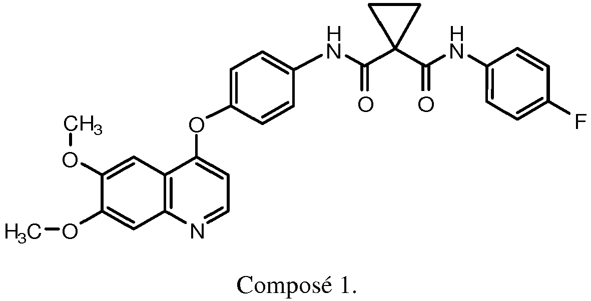

5. Composé destiné à une utilisation selon la revendication 4, le cancer médié par *KIF5B-RET* étant un adénocarcinome pulmonaire.

6. Composé destiné à une utilisation selon la revendication 5, l'adénocarcinome pulmonaire étant un cancer du poumon non à petites cellules.

7. Composé destiné à une utilisation selon la revendication 6, l'adénocarcinome pulmonaire étant un cancer du poumon non à petites cellules positif pour le gène de fusion *KIF5B-RET.*

8. Composé destiné à une utilisation selon la revendication 4, le composé étant administré sous la forme d'une composition pharmaceutique comprenant le composé et au moins un support pharmaceutiquement acceptable.

9. Composé destiné à une utilisation selon la revendication 8, la composition pharmaceutique étant administrée quotidiennement pendant plus de 3 mois.

10. Composé destiné à une utilisation selon la revendication 4, dans lequel, pour la détection du cancer du poumon non à petites cellules positif pour le gène de fusion *KIF5B-RET,* on utilise une épreuve FISH, CISH ou SISH.

11. Composé destiné à une utilisation selon la revendication 4, dans lequel, pour la détection du cancer du poumon non à petites cellules positif pour le gène de fusion *KIF5B-RET,* on utilise n'importe quelle forme de PCR génomique, de séquençage direct, de séquençage PCR, de RT-PCR, ou d'épreuve similaire.

12. Composé destiné à une utilisation selon l'une quelconque des revendications 1 à 11, le composé produisant au moins un effet thérapeutique sélectionné dans le groupe constitué d'une réduction de la taille d'une tumeur, d'une réduction du processus métastasique, d'une rémission complète, d'une rémission partielle, d'une maladie stable, d'une augmentation du taux de réponse global, et d'une réponse pathologique complète.

13. Composé destiné à une utilisation dans une méthode de diagnostic et de traitement d'un patient, le patient présentant une tumeur de type CPNPC et la tumeur étant identifiée comme un CPNPC positif pour le gène de fusion *KIF5B-RET,* le composé étant le sel malate du Composé 1 tel que défini dans la revendication 1, et le composé étant administré avec au moins un support pharmaceutiquement acceptable.

14. Composé destiné à une utilisation selon l'une quelconque des revendications 1 à 13, le Composé 1 étant administré par voie orale une fois par jour sous la forme du sel malate conditionné en capsule ou en comprimé contenant 20, 40, ou 60 mg de Composé 1.

15. Composé destiné à une utilisation selon l'une quelconque des revendications 1 à 14, le composé étant administré après un traitement par cisplatine et/ou gemcitabine et/ou docétaxel.
